# EUROPEAN PATENT APPLICATION

(11) **EP 4 261 910 A1**
(43) Date of publication of application: **18.10.2023**
(21) Application number: 21903466.7
(22) Date of filing: 09.12.2021
(51) Int. Cl.: H01L 51/50, C07D 487/04, C09K 11/06

(54) **MATERIAL FOR ORGANIC ELECTROLUMINESCENT ELEMENT AND ORGANIC ELECTROLUMINESCENT ELEMENT**

(30) Priority: 11.12.2020 JP 2020206133
(71) Applicant: NIPPON STEEL Chemical & Material Co., Ltd., Tokyo 103-0027 (JP)
(72) Inventor: KITAHARA, Ikumi, Tokyo 103-0027 (JP); HAYASHI, Kentaro, Tokyo 103-0027 (JP); OGAWA, Junya, Tokyo 103-0027 (JP); UEDA, Tokiko, Tokyo 103-0027 (JP)
(74) Representative: Vossius & Partner Patentanwälte Rechtsanwälte mbB
(86) International application number: PCT/JP2021/045321
(87) International publication number: WO 2022/124367

(57) **Abstract**

To provide a compound for an organic EL device which provides an organic EL device having a low driving voltage and also high efficiency and extended lifetime characteristics, and an organic EL device using the compound. The compound for an organic EL device is a compound having an indolocarbazole skeleton represented by the following general formula (1): 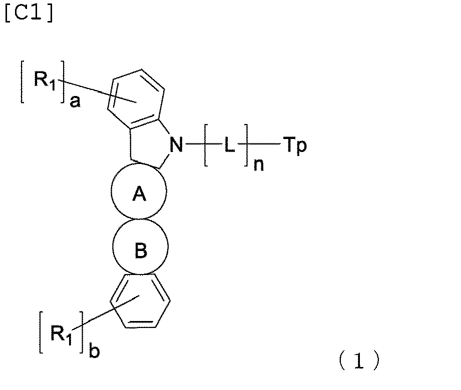 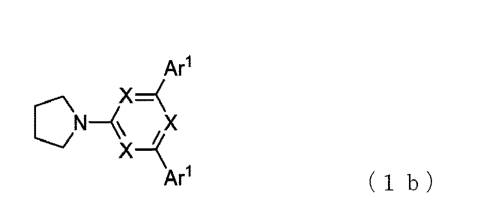 wherein a ring A is a benzene ring, a ring B is represented by formula (1b), Tp represents a triphenylene group, L represents an aromatic hydrocarbon group having 6 to 18 carbon atoms, X represents N or C-H and at least one thereof represents N, and n represents an integer of 1 to 3.

## Description

### Technical Field

The present invention relates to a compound for an organic electroluminescent element or device (organic EL device) and an organic EL device comprising a specific mixed host material.

### Background Art

Application of a voltage to an organic EL device allows injection of holes and electrons from an anode and a cathode, respectively, into a light-emitting layer. Then, in the light-emitting layer, injected holes and electrons recombine to generate excitons. At this time, according to statistical rules of electron spins, singlet excitons and triplet excitons are generated at a ratio of 1:3. Regarding a fluorescence-emitting organic EL device using light emission from singlet excitons, it is said that the internal quantum efficiency thereof has a limit of 25%. Meanwhile, regarding a phosphorescent organic EL device using light emission from triplet excitons, it is known that intersystem crossing is efficiently performed from singlet excitons, the internal quantum efficiency is enhanced to 100%.

Highly efficient organic EL devices utilizing delayed fluorescence have been developed recently. For example, Patent Literature 1 discloses an organic EL device utilizing a TTF (Triplet-Triplet Fusion) mechanism, which is one of delayed fluorescence mechanisms. The TTF mechanism utilizes a phenomenon in which singlet excitons are generated due to collision of two triplet excitons, and it is thought that the internal quantum efficiency can be theoretically raised to 40%. However, since the efficiency is lower compared to phosphorescent organic EL devices, further improvement in efficiency and low voltage characteristics are required.

In addition, Patent Literature 2 discloses an organic EL device utilizing a TADF (Thermally Activated Delayed Fluorescence) mechanism. The TADF mechanism utilizes a phenomenon in which reverse intersystem crossing from triplet excitons to singlet excitons is generated in a material having a small energy difference between a singlet level and a triplet level, and it is thought that the internal quantum efficiency can be theoretically raised to 100%.

However, all the mechanisms have room for advancement in terms of both efficiency and lifetime, and are additionally required to be improved also in terms of reduction in driving voltage.

### Citation List

### Patent Literature

Patent Literature 1: WO2010/134350 A
Patent Literature 2: WO2011/070963 A
Patent Literature 3: WO2008/056746 A
Patent Literature 4: WO2008/146839 A
Patent Literature 5: WO2013/056776 A
Patent Literature 6: JP2012-140365 A
Patent Literature 7: KR2019/0069083 A
Patent Literature 8: US2015/0171357 A
Patent Literature 9: WO2012/039561 A

Patent Literatures 3 and 4 disclose use of an indolocarbazole compound as a host material. Patent Literature 5 discloses use of a compound in which indenocarbazole is substituted with triphenylene and a nitrogen-containing six-membered ring, in a light-emitting layer. Patent Literatures 6, 7, 8, and 9 disclose use of a compound in which triphenylene is substituted with indolocarbazole.

However, none of these can be said to be sufficient, and further improvements in efficiency and lifetime of an organic EL device are desired.

### Summary of Invention

In order to apply organic EL devices to display devices such as flat panel displays, it is necessary to improve the luminous efficiency of devices and at the same time, to sufficiently secure the extended lifetime characteristics of the devices. In view of the above circumstances, an object of the present invention is to provide an organic EL device having a low driving voltage and also high efficiency and the extended lifetime characteristics, and a compound suitable therefor.

As a result of intensive studies, the present inventors have found that an organic EL device exhibits excellent characteristics by using a specific indolocarbazole compound, and have completed the present invention.

The present invention relates to a compound for an organic EL device, represented by the following general formula (1).

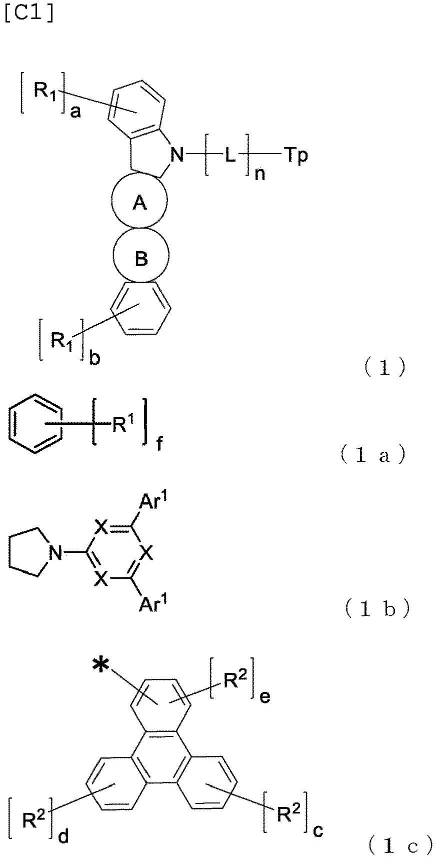

In the general formula (1), a ring A is an aromatic ring represented by formula (1a) and is fused to an adjacent ring.

A ring B is a five-membered heterocyclic ring represented by formula (1b) and is fused to an adjacent ring.

Tp represents a triphenylene group represented by formula (1c) and ^{∗}represents a position for binding to L.

Each R¹ independently represents deuterium or an aliphatic hydrocarbon group having 1 to 10 carbon atoms.

L represents a divalent substituted or unsubstituted aromatic hydrocarbon group having 6 to 18 carbon atoms.

Each X independently represents N or C-H and at least one X represents N.

Ar¹ each independently represent hydrogen, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic rings are linked to each other.

Each R² independently represents deuterium, an aliphatic hydrocarbon group having 1 to 10 carbon atoms, a substituted or unsubstituted aromatic hydrocarbon group having 6 to 10 carbon atoms, a substituted or unsubstituted aromatic heterocyclic group having 3 to 12 carbon atoms, or a substituted or unsubstituted linked aromatic group in which two to five aromatic rings of aromatic groups selected from the aromatic hydrocarbon group and the aromatic heterocyclic group are linked to each other.
a to f represent the number of substitutions, a to d represent an integer of 0 to 4, e represents an integer of 0 to 3, f represents an integer of 0 to 2, and n represents the number of repetitions and an integer of 1 to 3.

In preferred aspect of the present invention, any of all of X representing N, L representing a phenylene group, n representing 1, or all of a to f representing 0 is satisfied in the general formula (1).

In some aspects, the general formula (1) is represented by any one of the following formulas (2) to (5) : 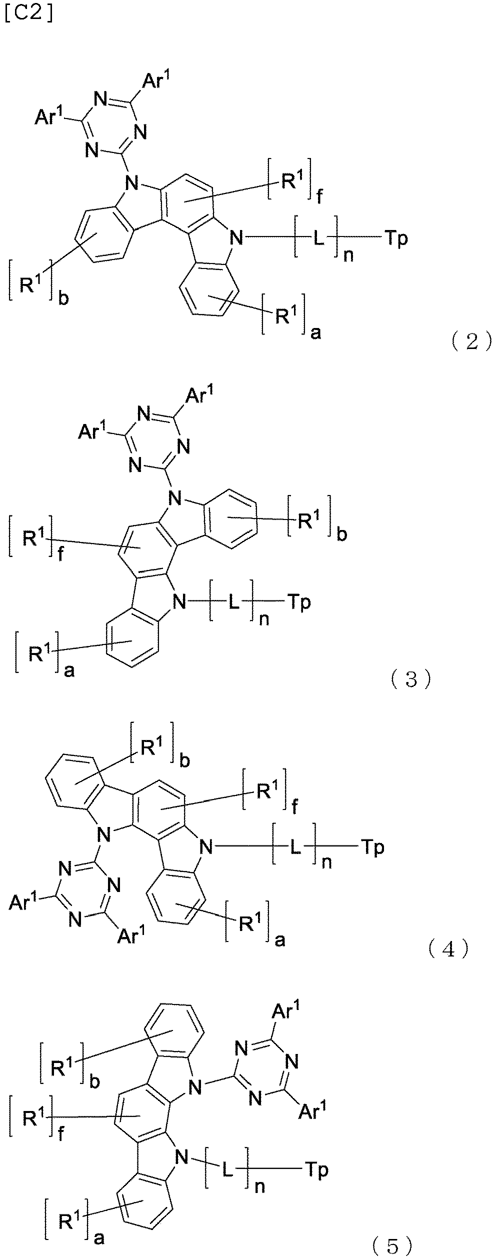 wherein Tp, Ar¹, L, R¹, R², a to f, and n are as defined for the general formula (1).

In some aspects, Tp is represented by the following formula (2c): 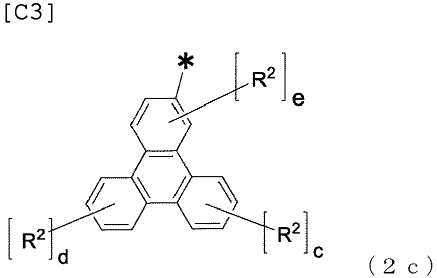 wherein R², c, d, e and * are as defined for the general formula (1).

The present invention relates to an organic EL device having an organic layer between an anode and a cathode laminated on a substrate, wherein the organic layer contains the above compound for an organic EL device.

The organic layer is, for example, a light-emitting layer, and preferably contains the compound for an organic EL device, as a host material.

In order to apply organic EL devices to display devices such as flat panel displays or light sources, it is necessary to improve the luminous efficiency of devices and at the same time, to extend the lifetime of devices. For improvement of the lifetime of devices, a higher durability of a material used for an organic layer against heat and charges is needed.

The compound for an organic EL device of the present invention has a nitrogen-containing six-membered ring high in electron transport properties, on one N in indolocarbazole assumed to have high hole transport properties and high stability against charges, and is substituted with triphenylene assumed to have stability against heat and charges, on another N in the indolocarbazole, and thus is considered to be capable of allowing extension of the lifetime of a device to be achieved, when used as a host material in an organic EL device.

Furthermore, it is assumed that, in the case of a delayed fluorescence-emitting EL device or a phosphorescent EL device, sufficiently high lowest excited triplet energy confining excitation energy generated in a light-emitting layer is possessed and a rigid triphenylene group is introduced to result in a reduction in loss of excitation energy due to molecular rotation or vibration, and it is assumed that an organic EL device can be unexpectedly obtained which causes no energy drain from a light-emitting layer toward any surrounding layer and which, while has a low voltage, has high efficiency and extended lifetime.

### Brief Description of Drawing

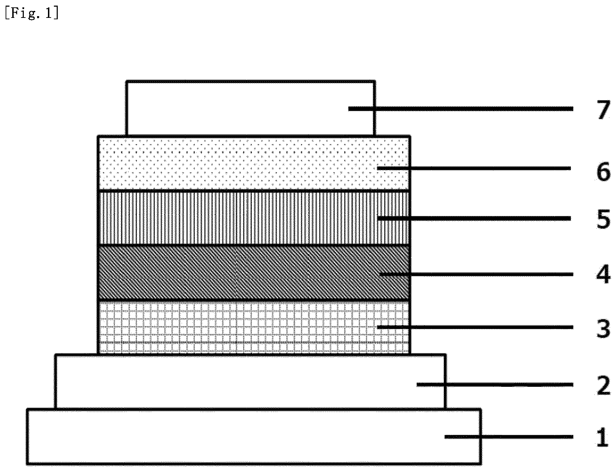

[Figure 1] Figure 1 is a schematic cross-sectional view showing one example of an organic EL device.

### Description of Embodiments

The compound for an organic EL device of the present invention is represented by the general formula (1). The compound for an organic EL device of the present invention is also referred to as the "material of the present invention" or "compound of the general formula (1)".

In the general formula (1), a ring A is a benzene ring represented by formula (1a), and is fused two adjacent rings.

A ring B is a five-membered heterocyclic ring represented by formula (1b), and is fused to two adjacent ring at any positions, but is not fused at a side containing N. Hence, an indolocarbazole ring has some isomeric structures, but the number of the structures is restricted.

Specifically, any structure represented by the formulas (2) to (5) can be contained. Preferred is any compound represented by the formulas (3) to (5).

In the general formula (1) and the formulas (2) to (5) and the formula (2c), the same symbols have the same meaning.

Each X independently represents C-H or N and at least one thereof represents N. Preferably, at least two X represent N, and more preferably, all of X represent N.
a to f represent the number of substitutions, a to d represent an integer of 0 to 4, e represents an integer of 0 to 3, and f represents an integer of 0 to 2. Preferably, a to f represent 0 to 1, more preferably 0.
n represents the number of repetitions, and an integer of 1 to 3, preferably 1 to 2, and more preferably 1.

Each Ar¹ independently represents hydrogen, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic rings are linked to each other. Preferred is hydrogen, a substituted or unsubstituted aromatic hydrocarbon group having 6 to 18 carbon atoms, or a substituted or unsubstituted linked aromatic group in which two to five of the aromatic hydrocarbon groups are linked to each other. More preferred is a substituted or unsubstituted phenyl group, or a substituted or unsubstituted linked aromatic group in which two to three phenyl groups are linked to each other.

Specific examples of the unsubstituted aromatic hydrocarbon group having 6 to 18 carbon atoms, the unsubstituted aromatic heterocyclic group having 3 to 17 carbon atoms, or the unsubstituted linked aromatic group in which two to five of these aromatic rings are linked to each other include a group generated by removing one hydrogen from benzene, naphthalene, acenaphthene, acenaphthylene, azulene, anthracene, chrysene, pyrene, phenanthrene, fluorene, triphenylene, pyridine, pyrimidine, triazine, thiophene, isothiazole, thiazole, pyridazine, pyrrole, pyrazole, imidazole, triazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzisothiazole, benzothiadiazole, purine, pyranone, coumarin, isocoumarin, chromone, dibenzofuran, dibenzothiophene, dibenzoselenophene, carbazole, or compounds in which two to five of these are linked to each other. Preferred examples thereof include a group generated from benzene, naphthalene, acenaphthene, acenaphthylene, azulene, anthracene, chrysene, pyrene, phenanthrene, fluorene, triphenylene, or compounds in which two to five of these are linked to each other. More preferred is a phenyl group, a biphenyl group, or a terphenyl group. The terphenyl group may be linked linearly or branched.

L represents a substituted or unsubstituted aromatic hydrocarbon group having 6 to 18 carbon atoms. Preferred is a substituted or unsubstituted phenylene group. When n represents 2 or 3, L has a linked structure and the binding style may be any of ortho-, meta-, or paralinking. Herein, the linked structure is a structure in which aromatic hydrocarbon rings are bound by a single bond.

When L represents an unsubstituted aromatic hydrocarbon group having 6 to 18 carbon atoms, the same as in the case of Ar¹ being an unsubstituted aromatic hydrocarbon group having 6 to 18 carbon atoms is applied, except that a divalent group generated by removing two hydrogen atoms from the aromatic hydrocarbon compound is adopted. Preferred is a phenylene group.

Each R¹ independently represents deuterium or an aliphatic hydrocarbon group having 1 to 10 carbon atoms.

Specific examples of the aliphatic hydrocarbon group having 1 to 10 carbon atoms include methyl, ethyl, propyl, butyl, pentyl, hexyl, heptyl, octyl, nonyl, and decyl. Preferred examples thereof include an alkyl group having 1 to 4 carbon atoms.

In the general formula (1), Tp represents a triphenylene group represented by formula (1c). Preferred is a triphenylene group represented by formula (2c) .

Each R² independently represents deuterium, an aliphatic hydrocarbon group having 1 to 10 carbon atoms, a substituted or unsubstituted aromatic hydrocarbon group having 6 to 10 carbon atoms, a substituted or unsubstituted aromatic heterocyclic group having 3 to 17 carbon atoms, or a substituted or unsubstituted linked aromatic group in which two to five of these aromatic rings are linked to each other, and preferred is deuterium, a substituted or unsubstituted phenyl group or a substituted or unsubstituted linked aromatic group in which two to five of these aromatic rings are linked to each other. Further preferred is a substituted or unsubstituted phenyl group or a linked aromatic group in which two to three of these aromatic rings are linked to each other.

Specific examples of the aliphatic hydrocarbon group having 1 to 10 carbon atoms are the same as in the case of R¹ being an aliphatic hydrocarbon group having 1 to 10 carbon atoms.

Specific examples of the unsubstituted aromatic hydrocarbon group having 6 to 10 carbon atoms, the unsubstituted aromatic heterocyclic group having 3 to 17 carbon atoms, or the unsubstituted linked aromatic group in which two to five of these aromatic rings are linked to each other are the same as in the case of Ar¹ being such a group.

Preferred examples include an aromatic group generated from benzene, pyridine, pyrimidine, triazine, thiophene, isothiazole, thiazole, pyridazine, pyrrole, pyrazole, imidazole, triazole, pyrazine, furan, isoxazole, oxazole, quinoline, isoquinoline, quinoxaline, quinazoline, oxadiazole, thiadiazole, benzotriazine, phthalazine, tetrazole, indole, benzofuran, benzothiophene, benzoxazole, benzothiazole, indazole, benzimidazole, benzotriazole, benzisothiazole, or benzothiadiazole. More preferred examples thereof include an aromatic group generated from benzene.

In the present specification, an aromatic hydrocarbon group, an aromatic heterocyclic group, or a linked aromatic group may each have a substituent. In the case of having a substituent, the substituent is preferably deuterium, halogen, a cyano group, a triarylsilyl group, an aliphatic hydrocarbon group having 1 to 10 carbon atoms, an alkenyl group having 2 to 5 carbon atoms, an alkoxy group having 1 to 5 carbon atoms, or a diarylamino group having 12 to 44 carbon atoms. In the case of the aliphatic hydrocarbon group having 1 to 10 carbon atoms, the substituent may be linear, branched, or cyclic. When the aromatic hydrocarbon group, the aromatic heterocyclic group, or the linked aromatic group is substituted with the triarylsilyl group or the diarylamino group, silicon and carbon, or nitrogen and carbon are each bound by a single bond.

Note that the number of substituents is 0 to 5 and preferably 0 to 2. When an aromatic hydrocarbon group and an aromatic heterocyclic group have substituents, the calculation of the number of carbon atoms does not include the number of carbon atoms of the substituents. However, it is preferred that the total number of carbon atoms including the number of carbon atoms of substituents satisfy the above range.

Specific examples of the substituent include cyano, methyl, ethyl, propyl, i-propyl, butyl, t-butyl, pentyl, cyclopentyl, hexyl, cyclohexyl, heptyl, octyl, nonyl, decyl, vinyl, propenyl, butenyl, pentenyl, methoxy, ethoxy, propoxy, butoxy, pentoxy, diphenylamino, naphthylphenylamino, dinaphthylamino, dianthranylamino, diphenanthrenylamino, and dipyrenylamino. Preferred examples thereof include cyano, methyl, ethyl, t-butyl, propyl, butyl, pentyl, hexyl, heptyl, or octyl,diphenylamino, naphthylphenylamino, or dinaphthylamino.

In the present specification, the linked aromatic group refers to an aromatic group in which the carbon atoms of the aromatic rings in aromatic groups selected from the aromatic hydrocarbon group and the aromatic heterocyclic group are linked to each other by a single bond. It is an aromatic group in which two or more aromatic groups are linked to each other, and they may be linear or branched. The aromatic group may be an aromatic hydrocarbon group or an aromatic heterocyclic group, and the plurality of aromatic groups may be the same or different. The aromatic group corresponding to the linked aromatic group is different from the substituted aromatic group.

Some or all hydrogen atoms of the unsubstituted aromatic hydrocarbon group, the unsubstituted aromatic heterocyclic group, the unsubstituted linked aromatic group, the substituents of these aromatic groups, or the aliphatic hydrocarbon group may be deuterated. Some or all hydrogen atoms in the general formula (1), the formulas (1a) to (1c), the formulas (2) to (5), and the formula (2c) may be deuterated.

Specific examples of the compounds represented by the general formula (1) are shown below, but are not limited to these exemplified compounds.

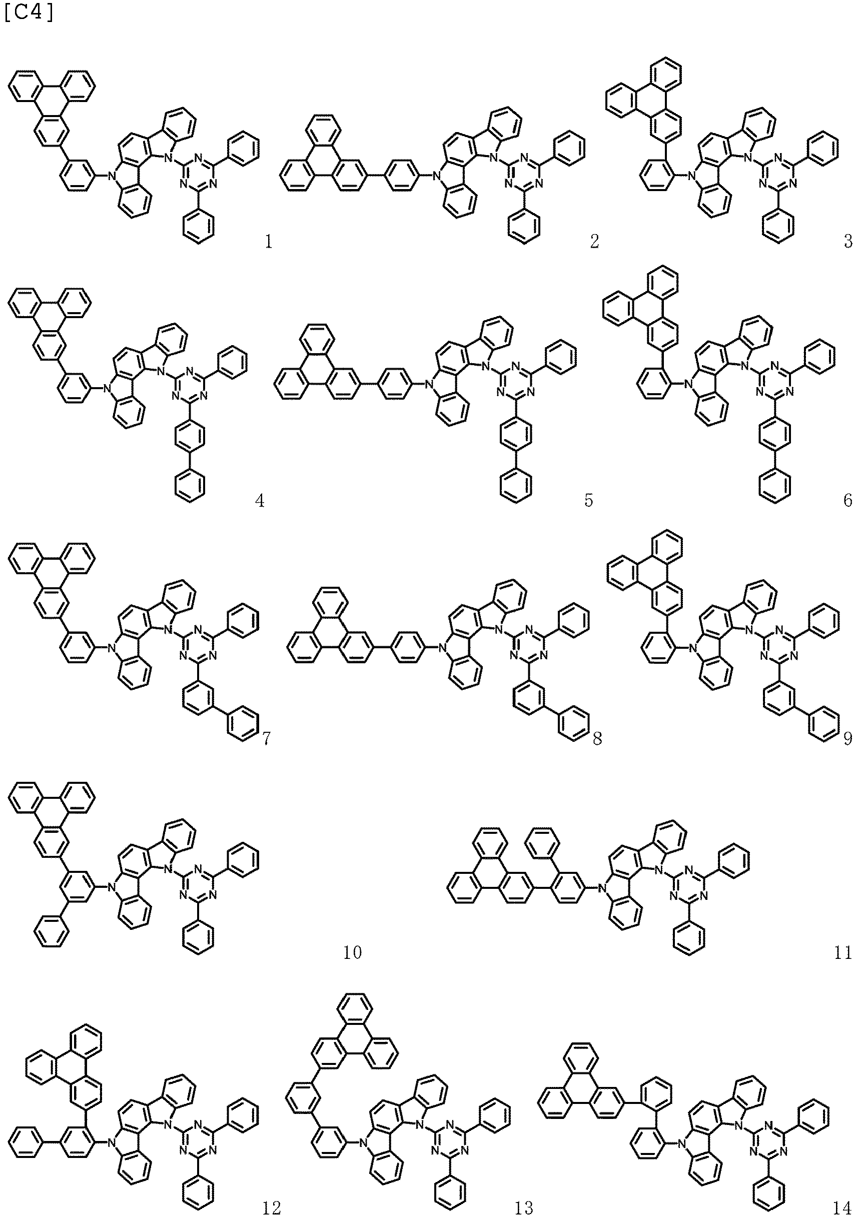

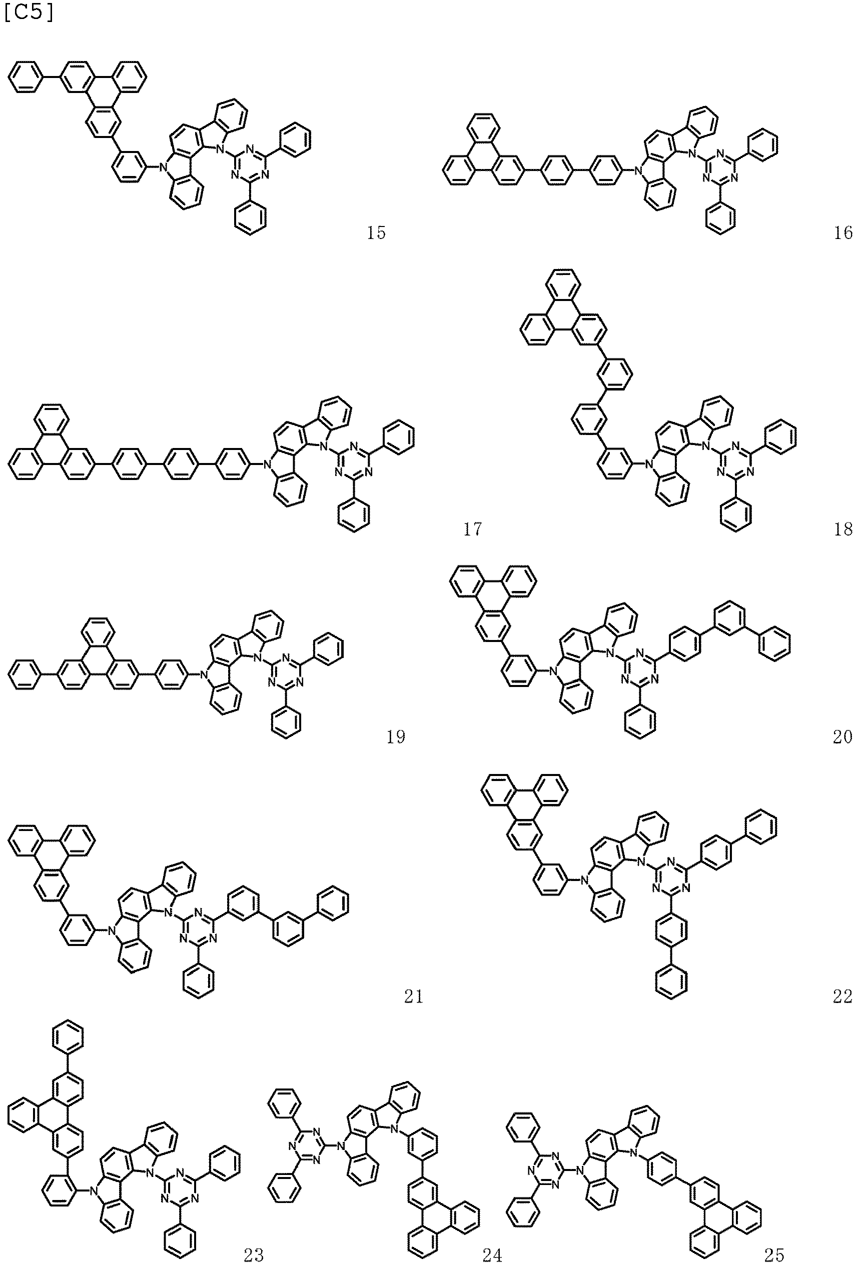

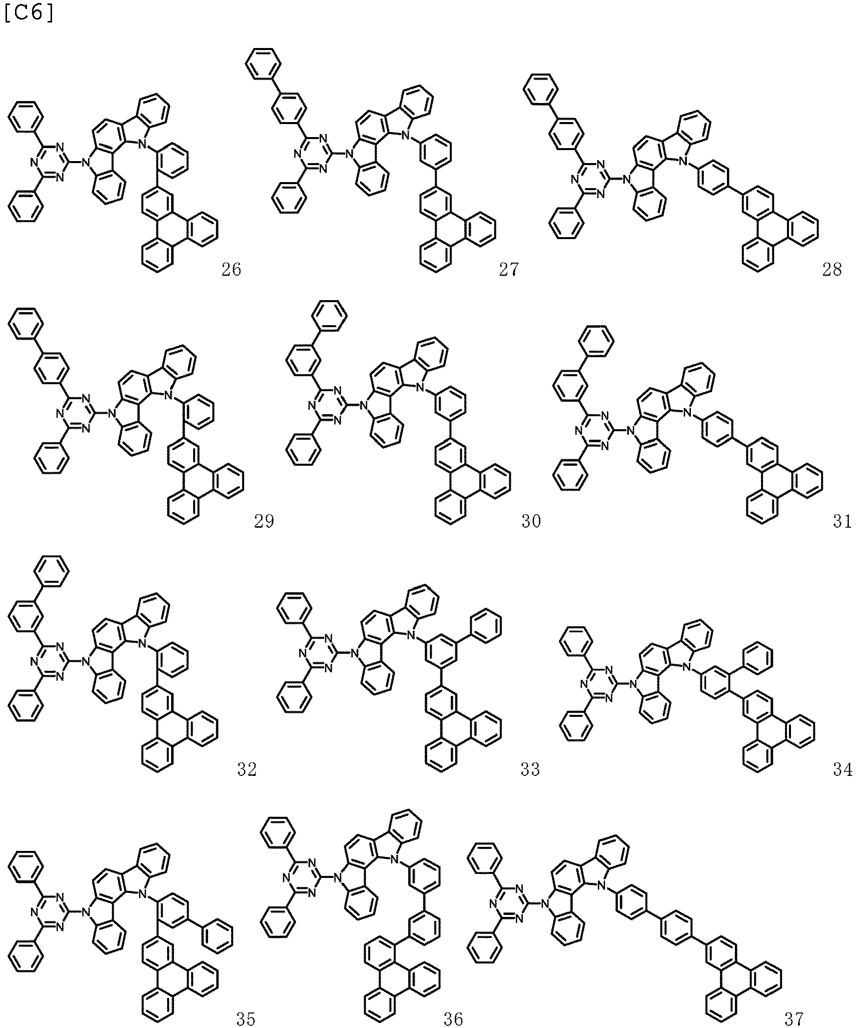

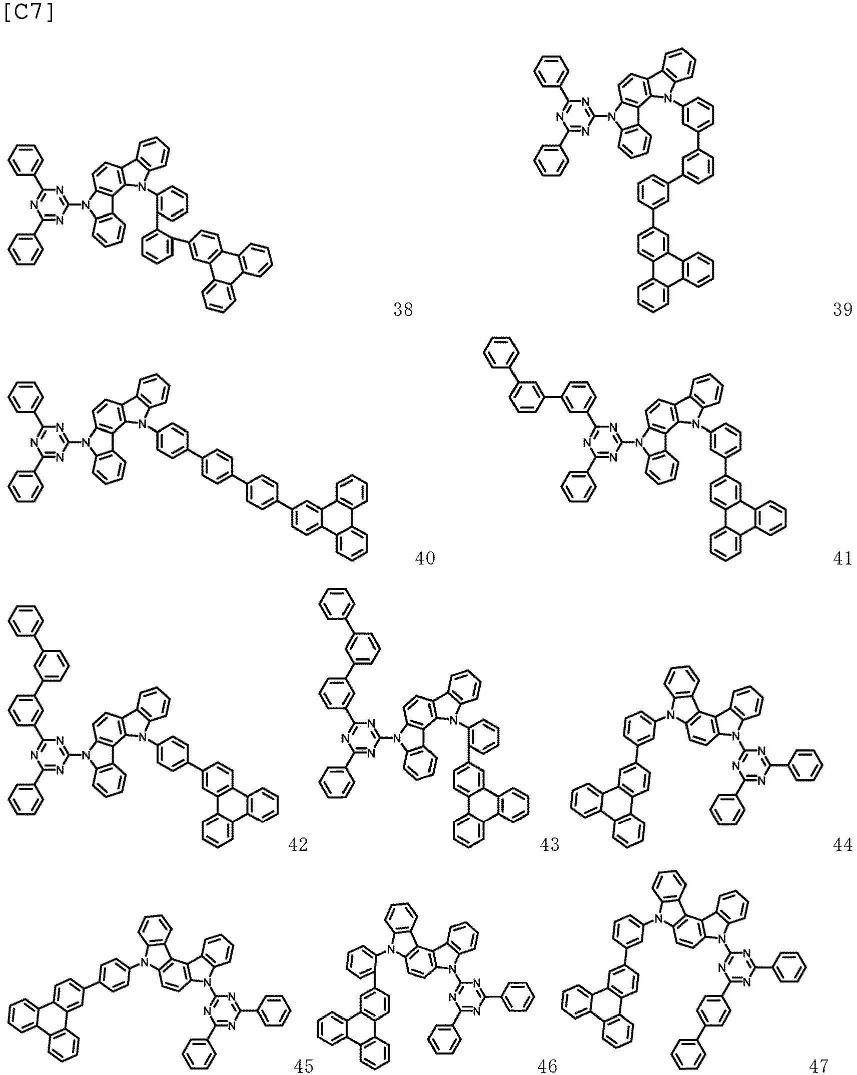

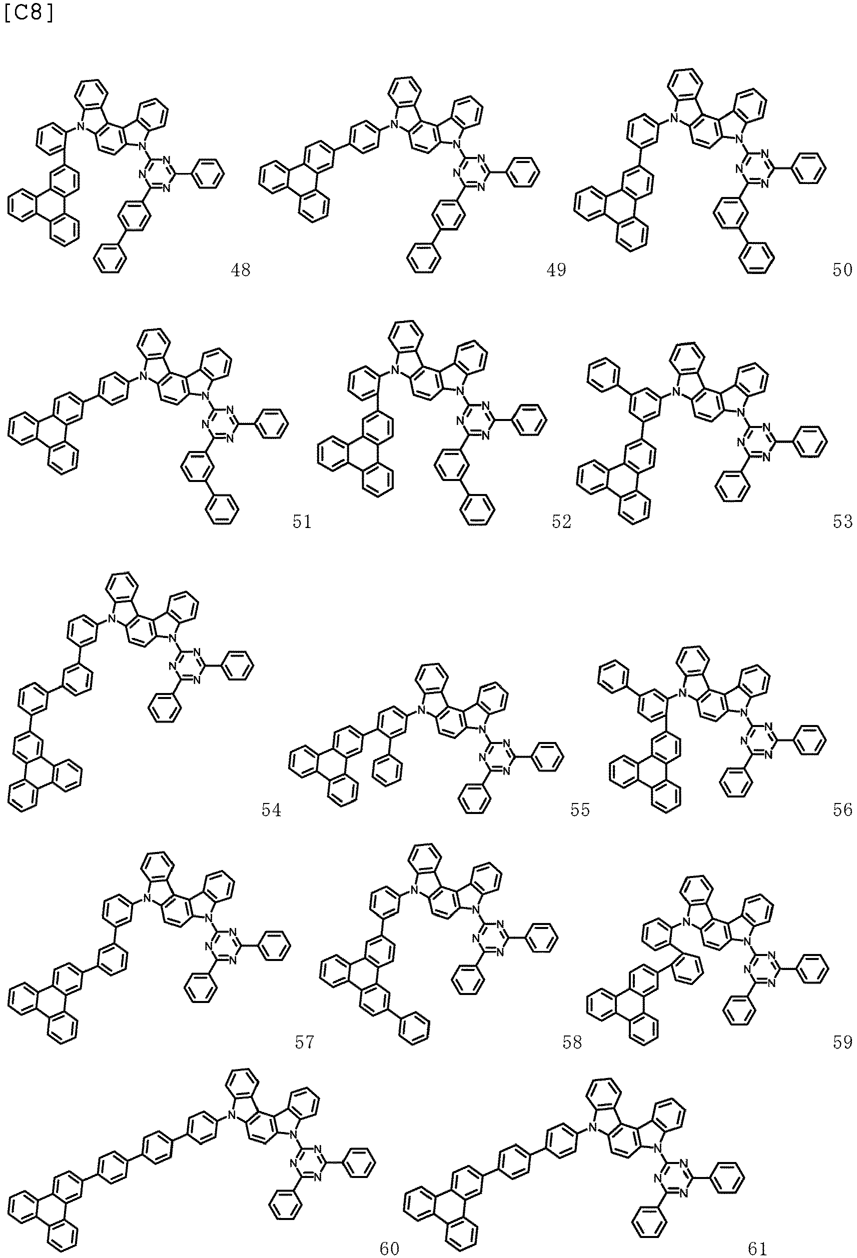

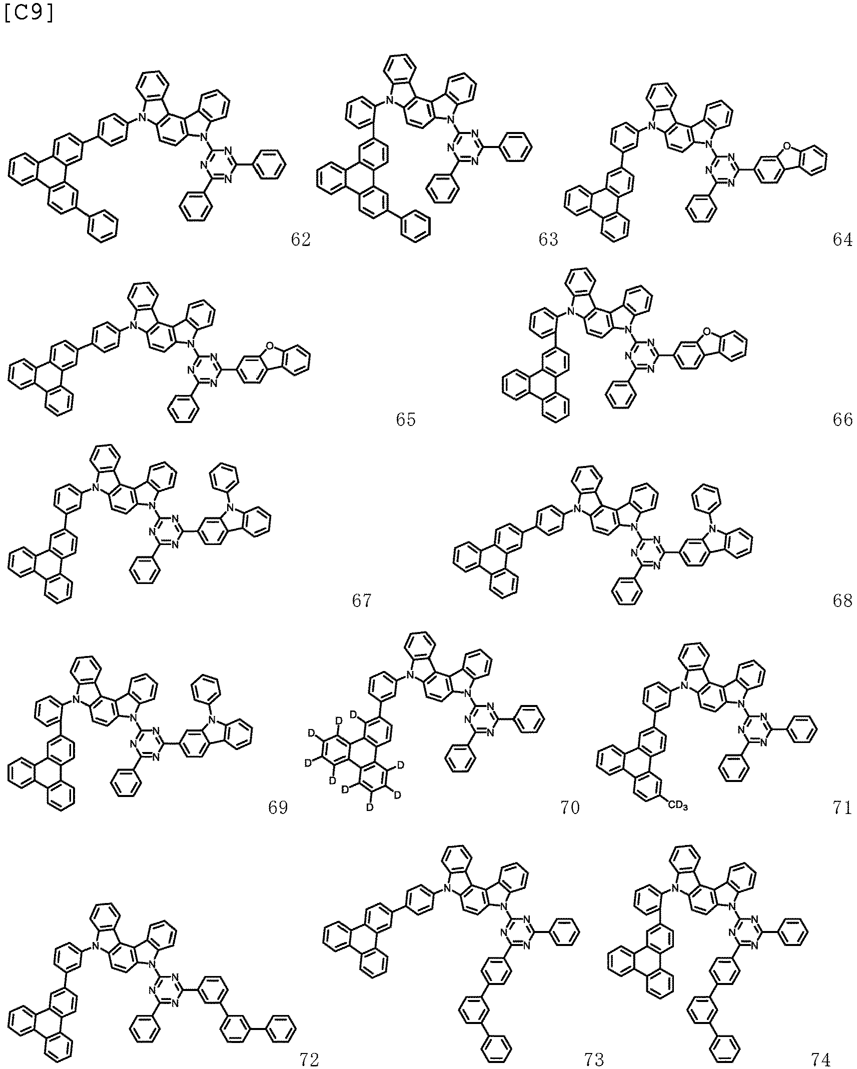

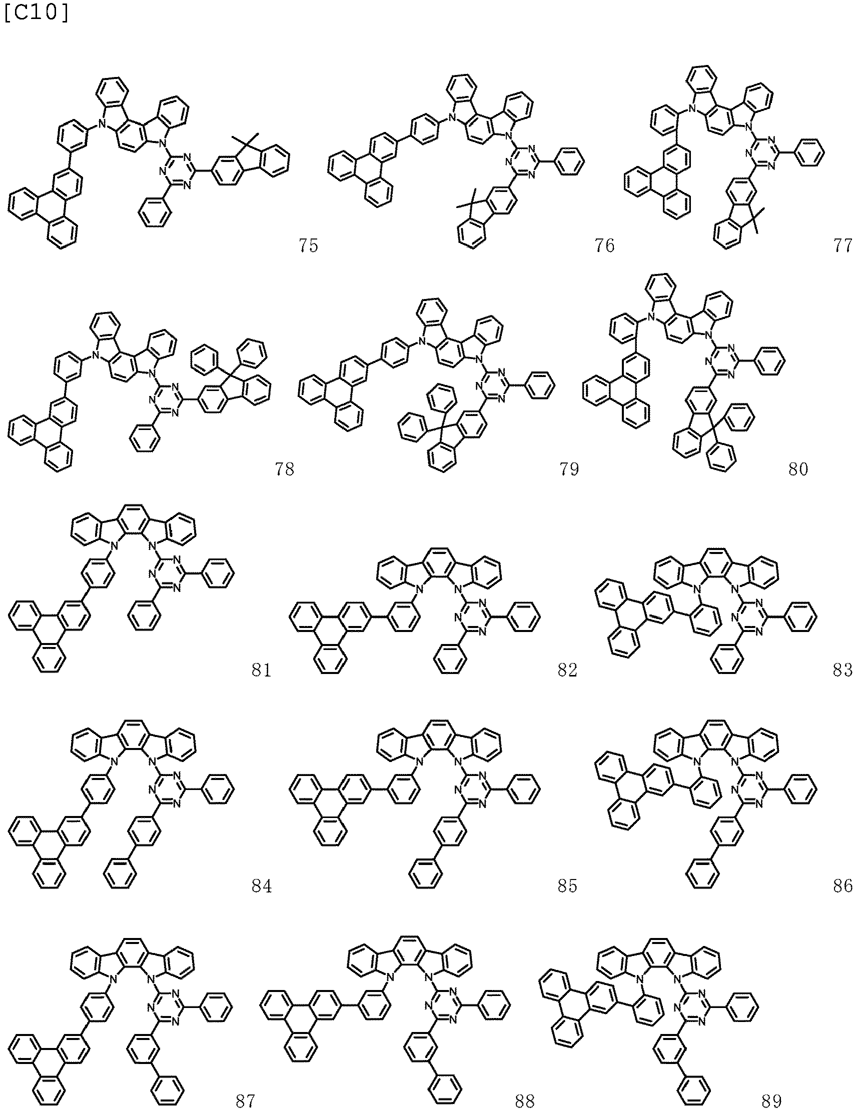

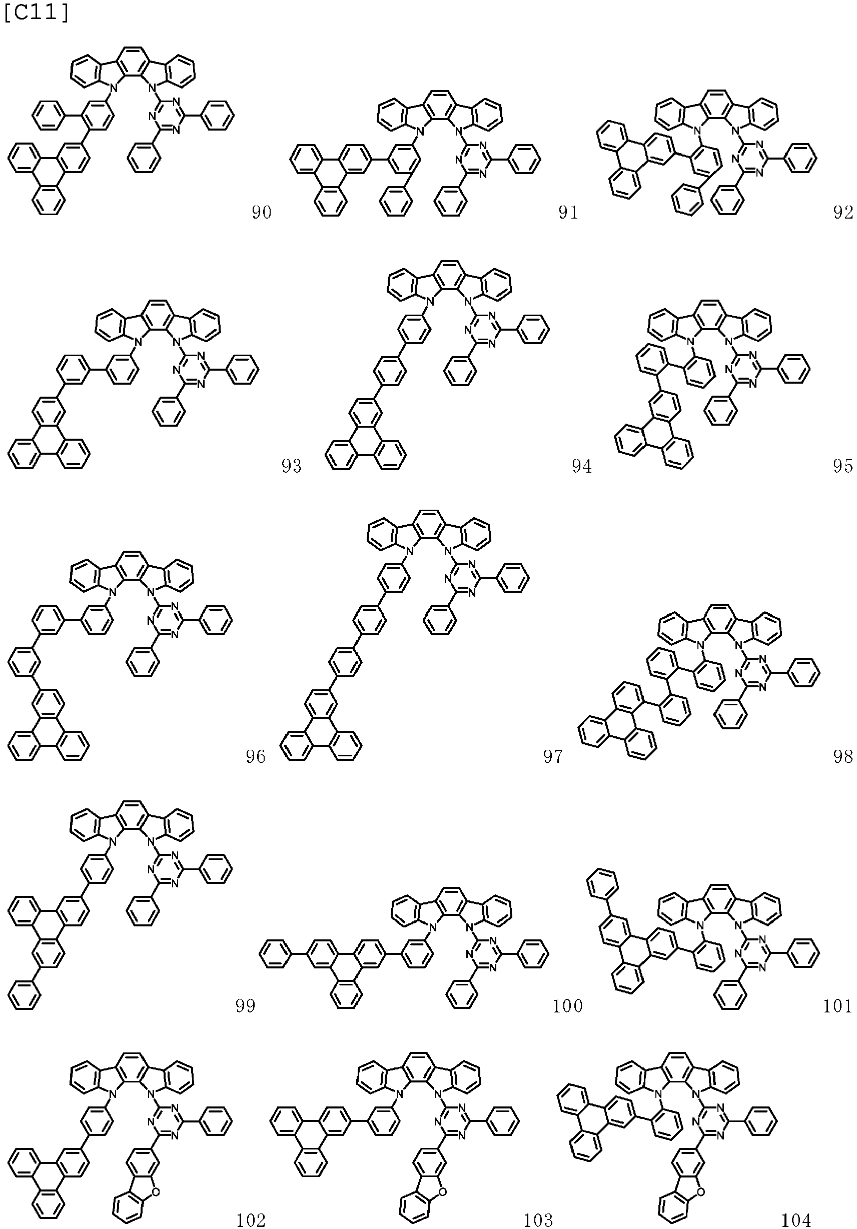

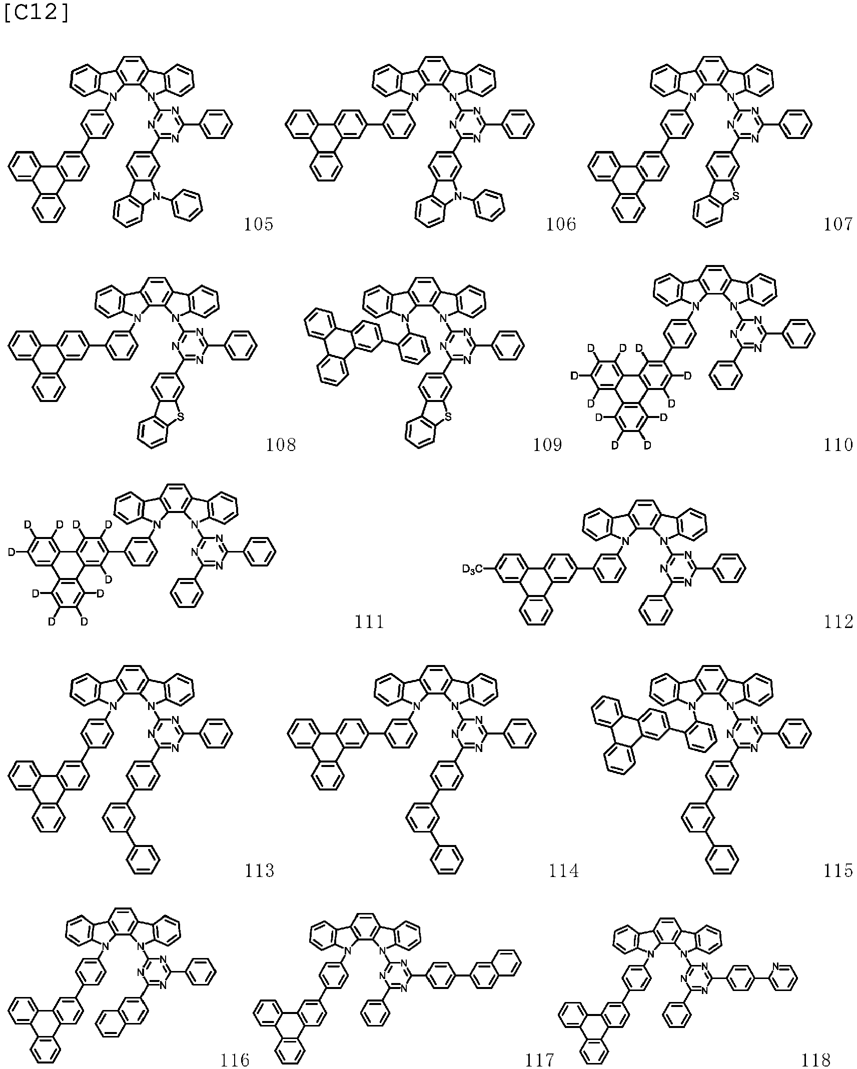

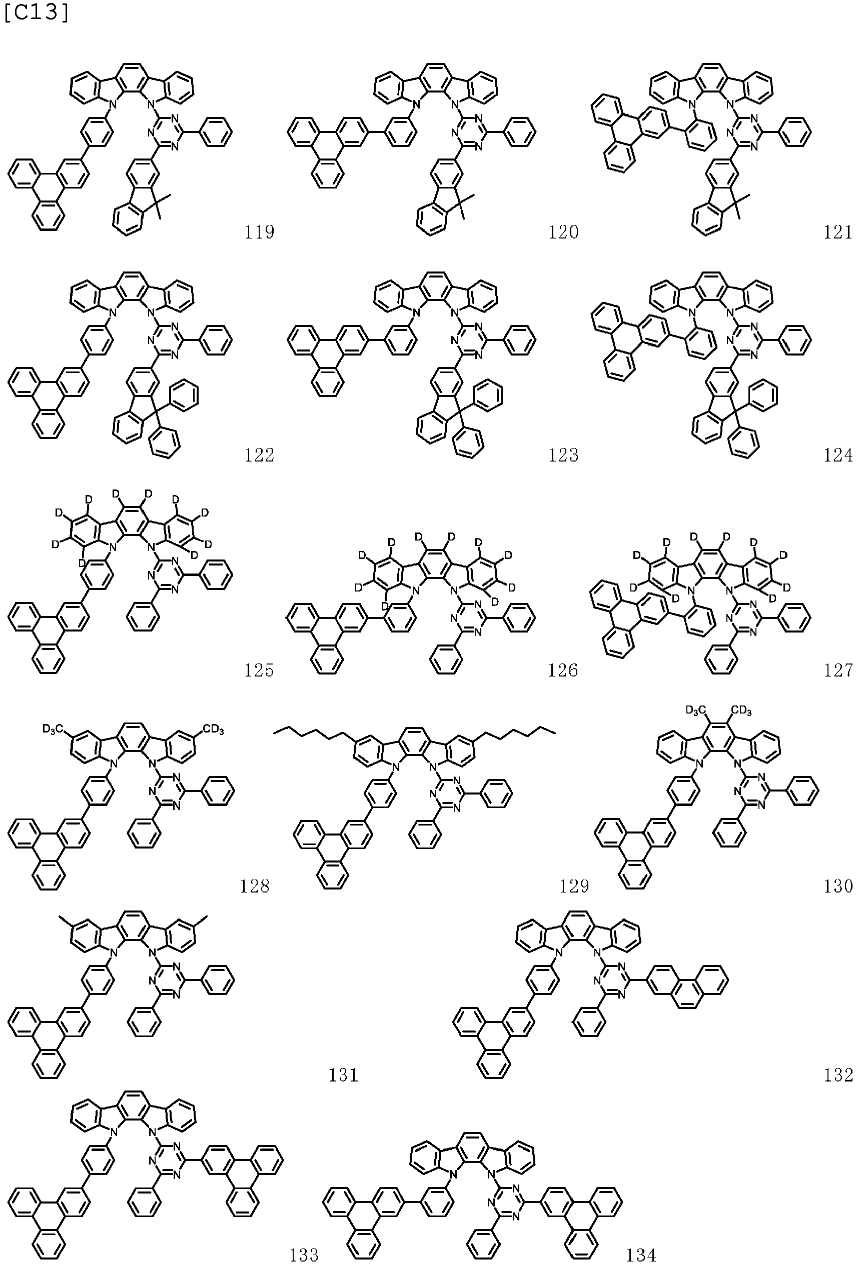

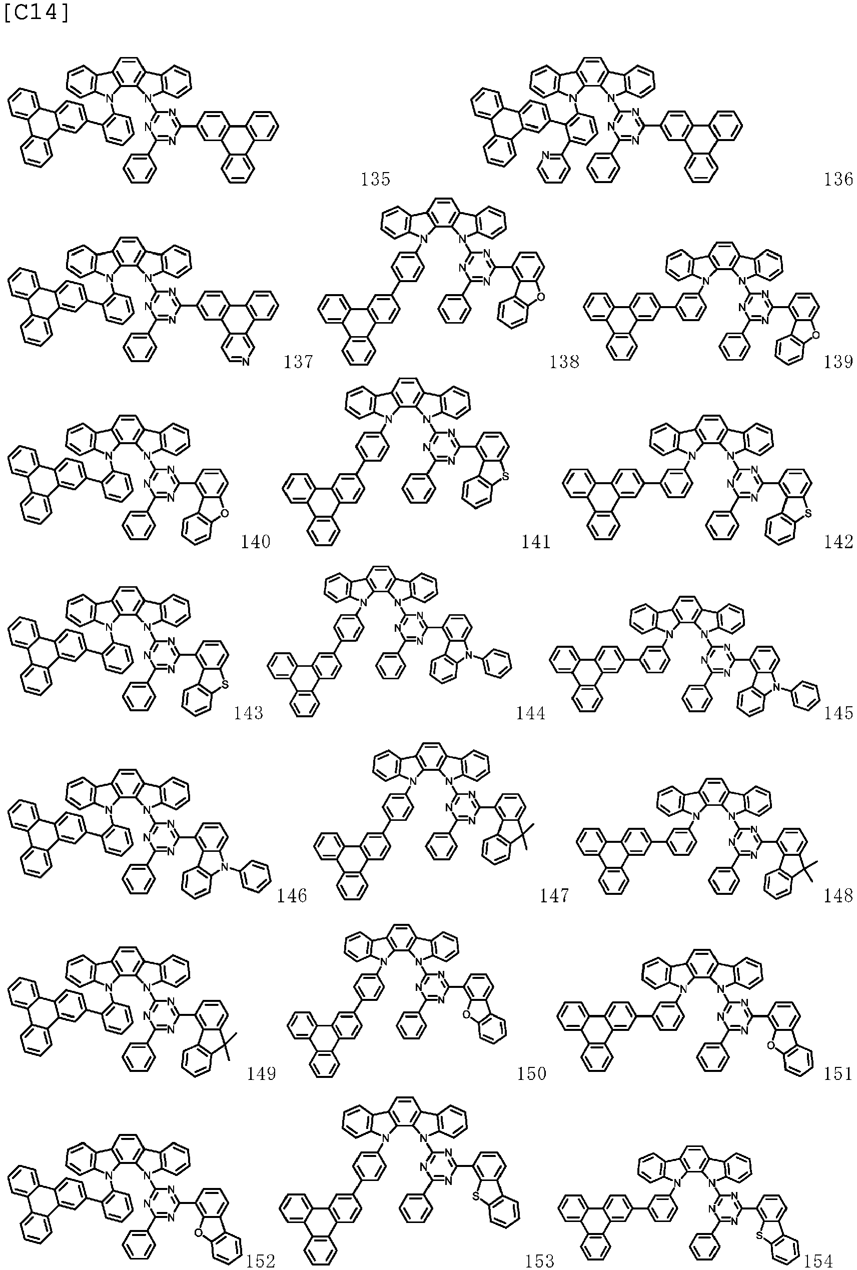

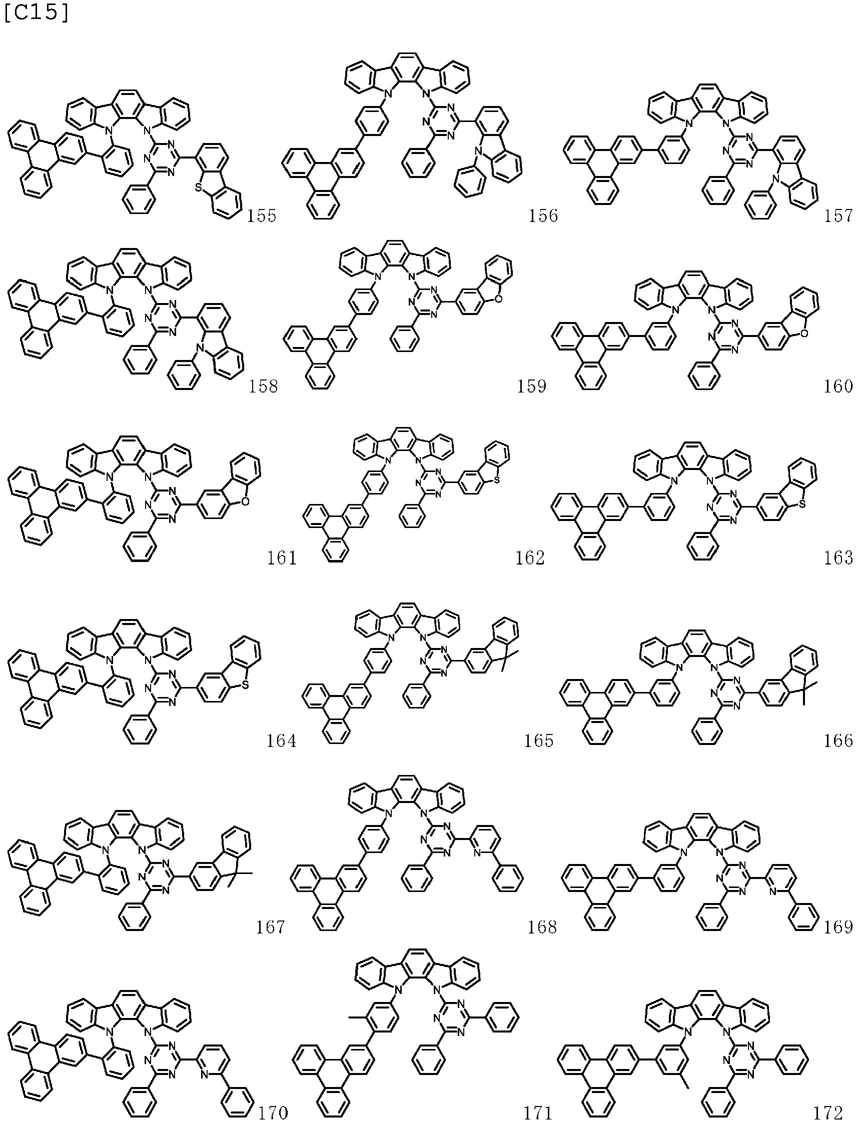

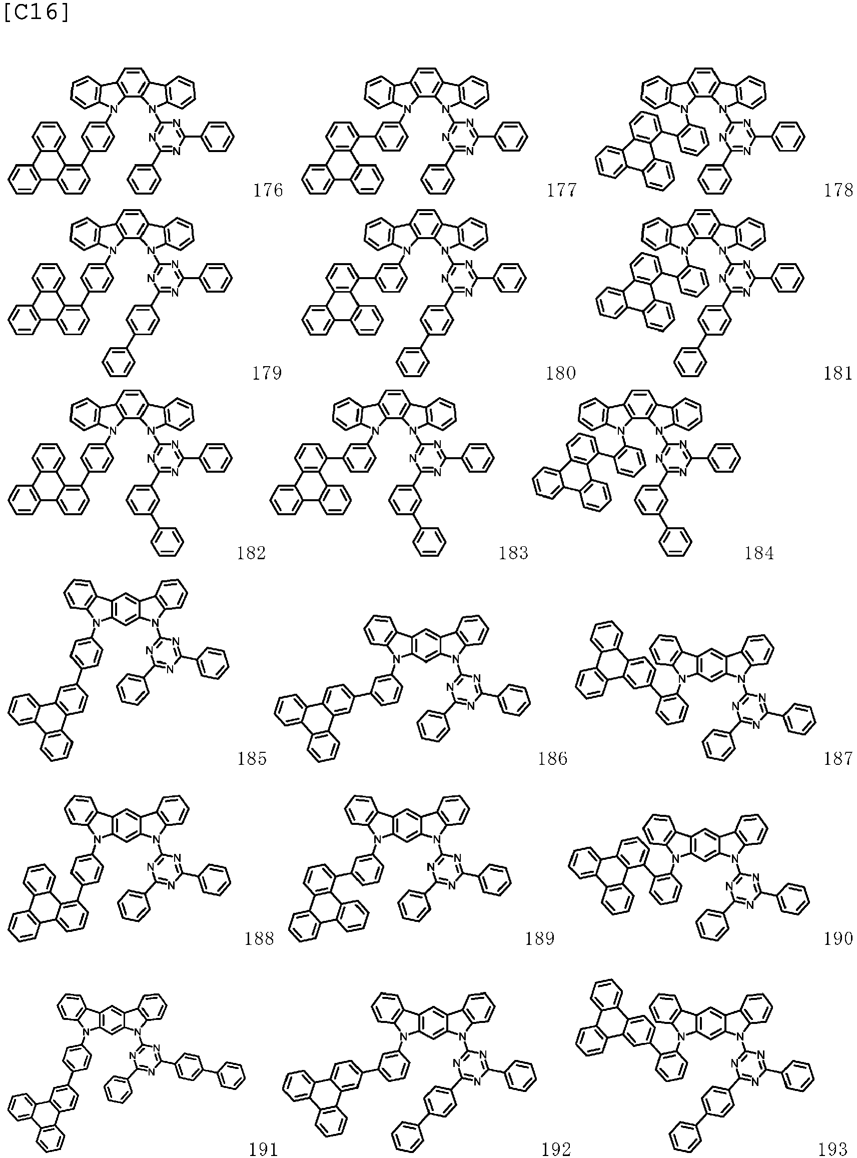

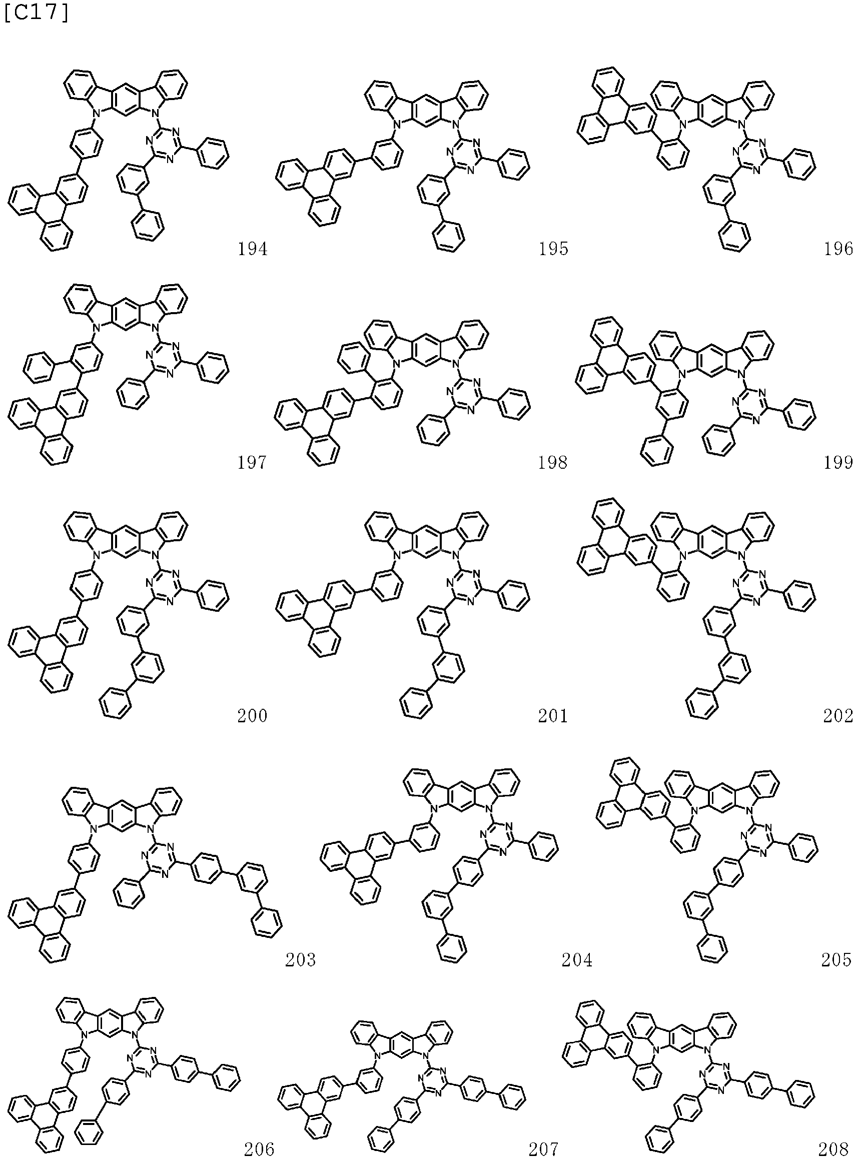

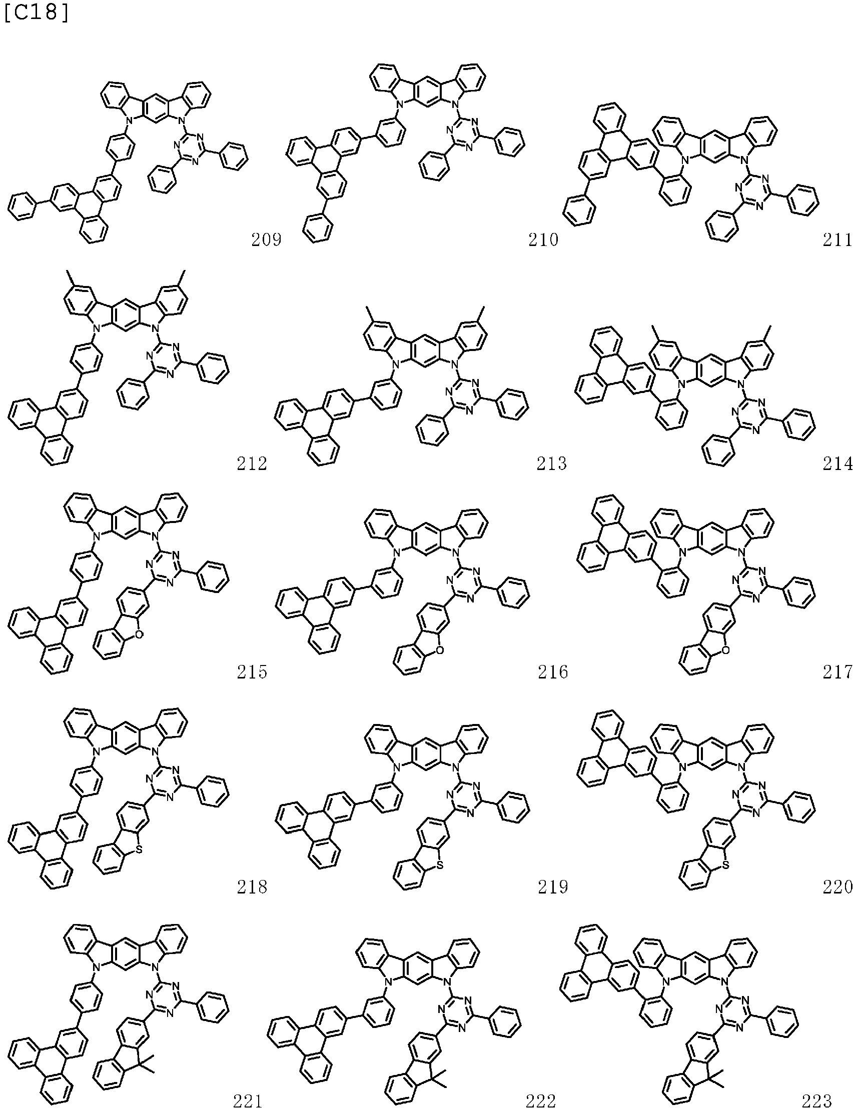

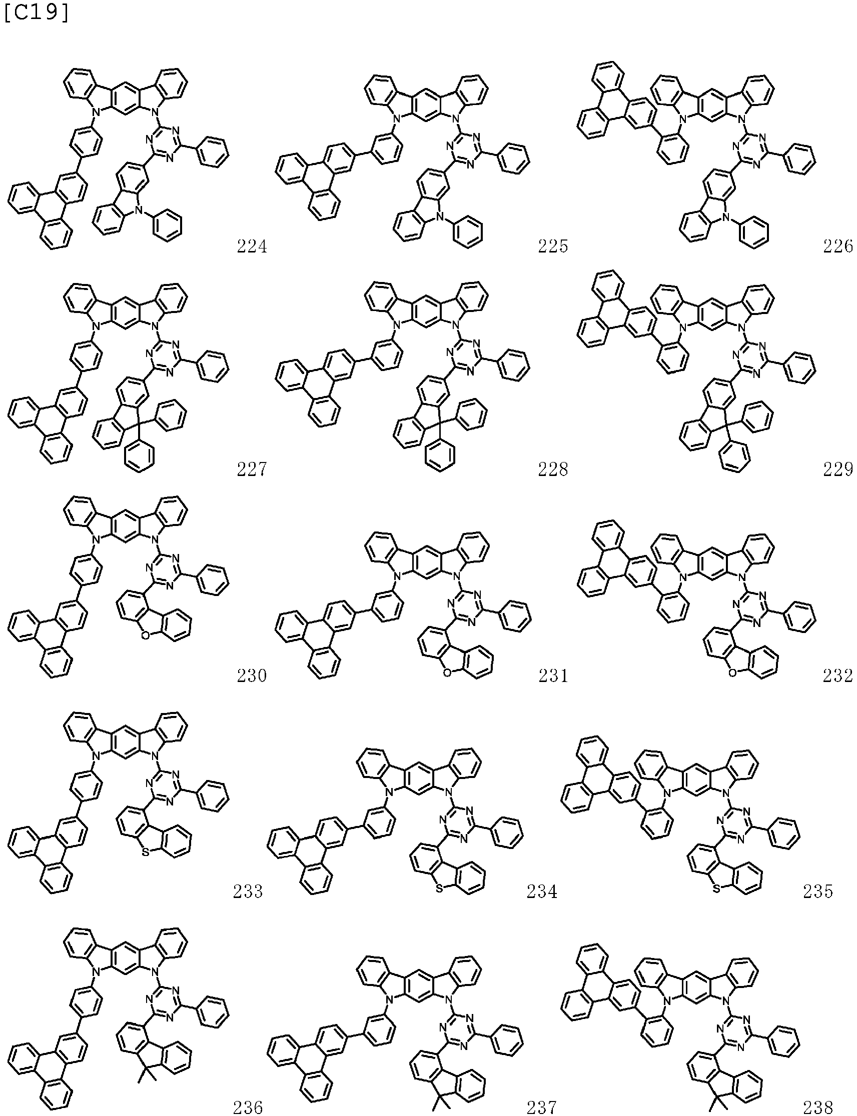

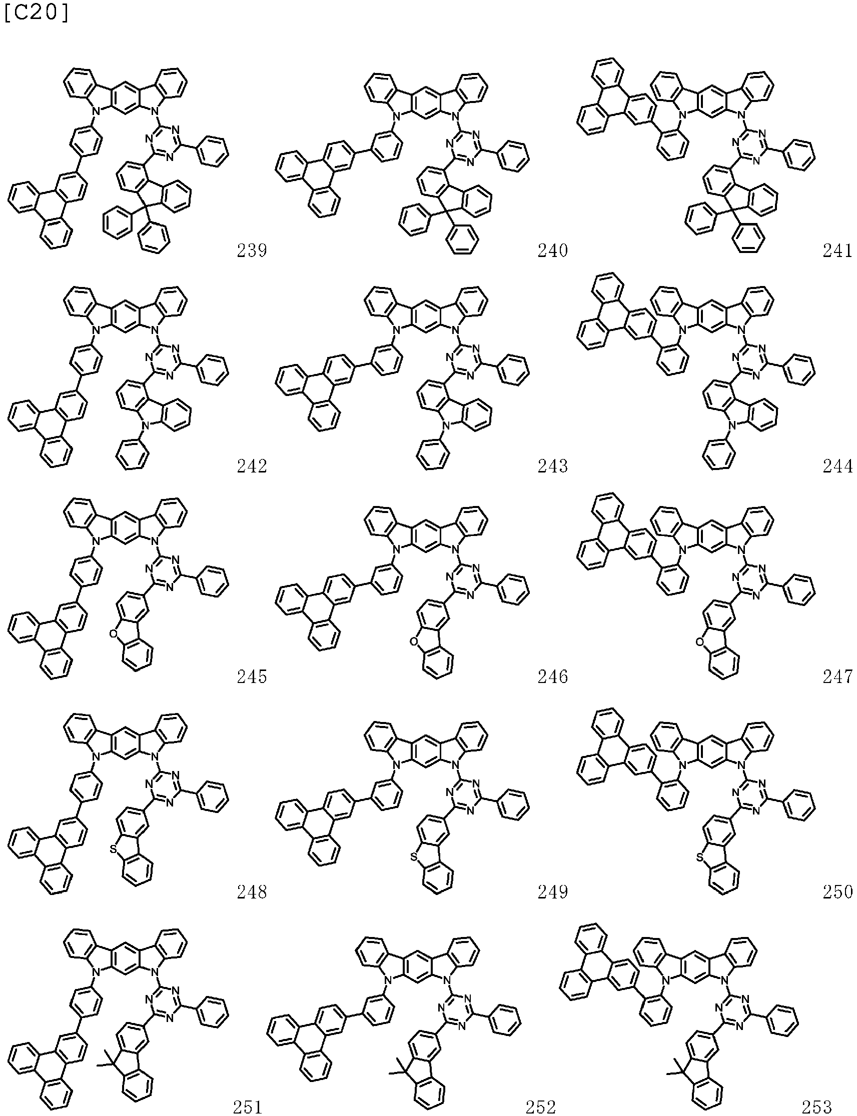

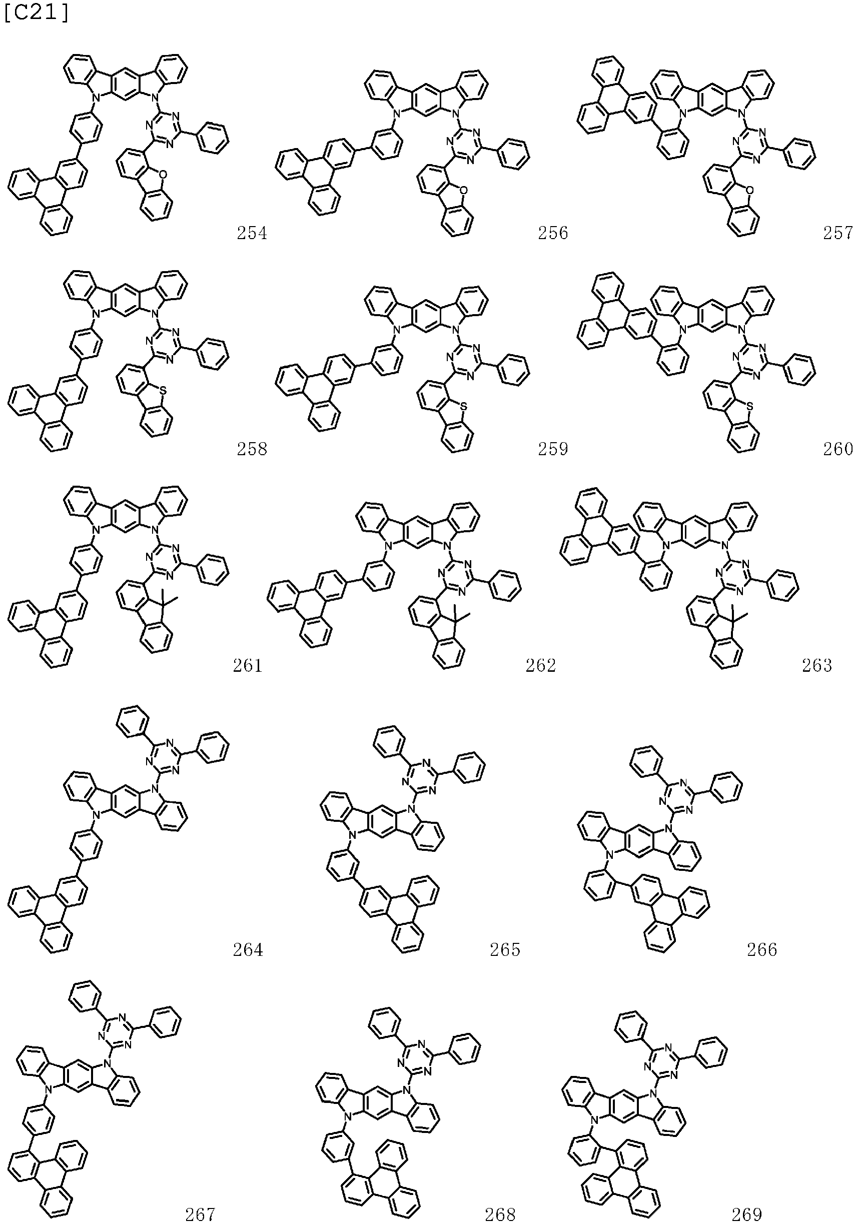

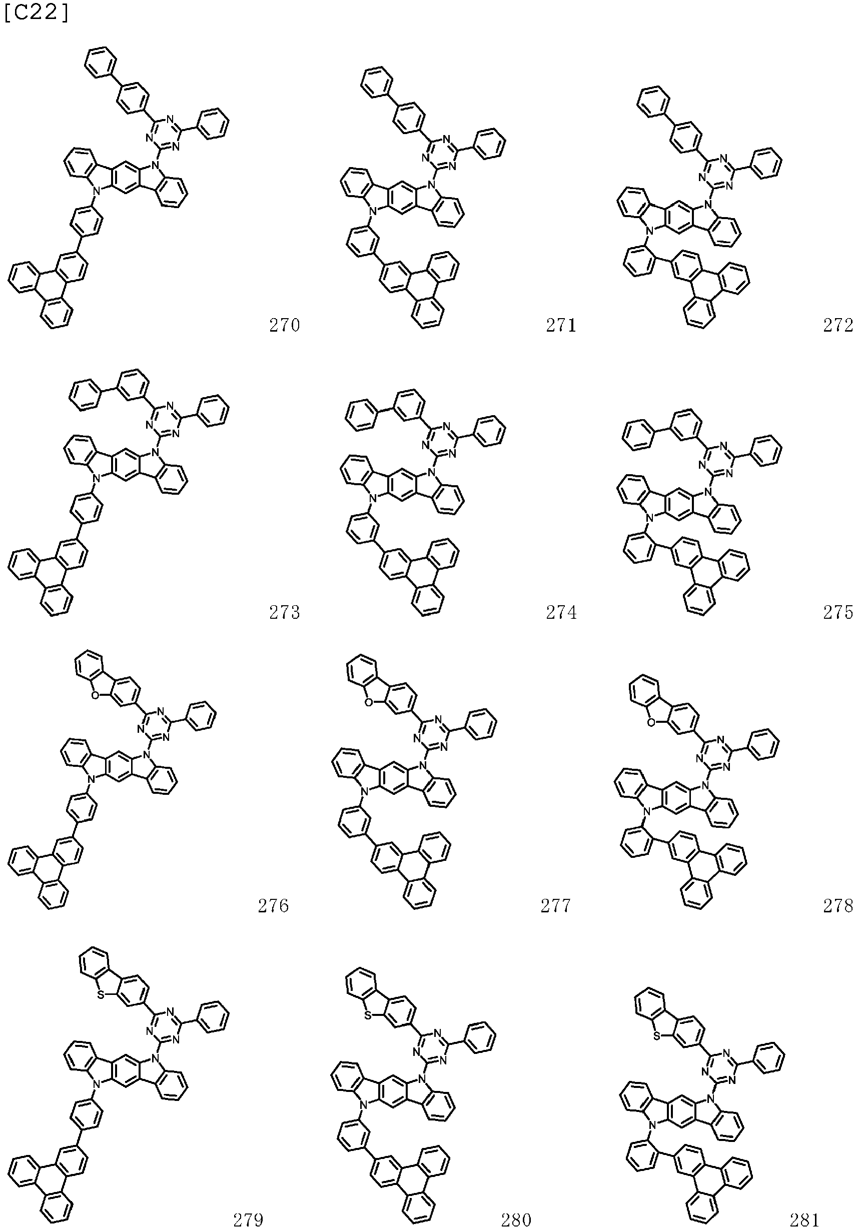

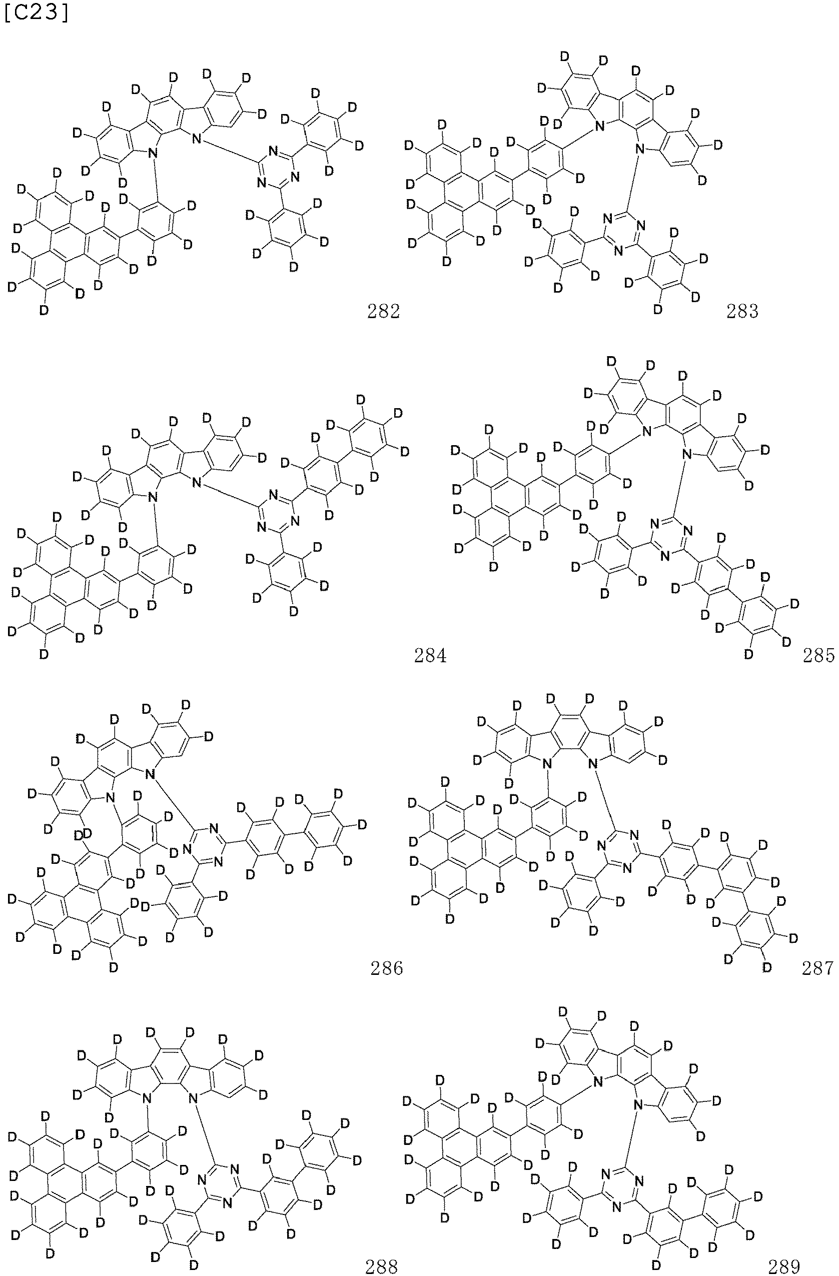

The compound for an organic EL device of the present invention is contained in an organic layer of an organic EL device, and this organic layer may be selected from the group consisting of a light-emitting layer, a hole injection layer, a hole transport layer, an electron transport layer, an electron injection layer, a hole blocking layer, and an electron blocking layer.

Preferred is a light-emitting layer, and the light-emitting layer may contain at least one light-emitting dopant.

If the compound for an organic EL device of the present invention is contained in the light-emitting layer, it is desirable that the material be contained as a host.

Next, the structure of the organic EL device of the present invention will be described by referring to the drawing, but the structure of the organic EL device of the present invention is not limited thereto.

Figure 1 is a cross-sectional view showing a structure example of an organic EL device generally used for the present invention, in which there are indicated a substrate 1, an anode 2, a hole injection layer 3, a hole transport layer 4, a light-emitting layer 5, an electron transport layer 6, and a cathode 7. The organic EL device of the present invention may have an exciton blocking layer adjacent to the light-emitting layer and may have an electron blocking layer between the light-emitting layer and the hole injection layer. The exciton blocking layer can be inserted into either of the anode side and the cathode side of the light-emitting layer and inserted into both sides at the same time. The organic EL device of the present invention has the anode, the light-emitting layer, and the cathode as essential layers, and preferably has a hole injection transport layer and an electron injection transport layer in addition to the essential layers, and further preferably has a hole blocking layer between the light-emitting layer and the electron injection transport layer. Note that the hole injection transport layer refers to either or both of a hole injection layer and a hole transport layer, and the electron injection transport layer refers to either or both of an electron injection layer and an electron transport layer.

A structure reverse to that of Figure 1 is applicable, in which a cathode 7, an electron transport layer 6, a light-emitting layer 5, a hole transport layer 4, and an anode 2 are laminated on a substrate 1 in this order. In this case, layers may be added or omitted as necessary.

### - Substrate -

The organic EL device of the present invention is preferably supported on a substrate. The substrate is not particularly limited, and those conventionally used in organic EL devices may be used, and substrates made of, for example, glass, a transparent plastic, or quartz may be used.

### - Anode -

Regarding an anode material for an organic EL device, it is preferable to use a material of a metal, an alloy, an electrically conductive compound, and a mixture thereof, each having a large work function (4 eV or more). Specific examples of such an electrode material include a metal such as Au, and a conductive transparent material such as CuI, indium tin oxide (ITO), SnO₂, and ZnO. In addition, an amorphous material such as IDIXO (In₂O₃-ZnO), which is capable of forming a transparent conductive film, may be used. Regarding the anode, such an electrode material is used to form a thin film by, for example, a vapor-deposition or sputtering method, and a desired shape pattern may be formed by a photolithographic method; or if the pattern accuracy is not particularly required (about 100 µm or more), a pattern may be formed via a desired shape mask when the electrode material is vapor-deposited or sputtered. Alternatively, when a coatable substance such as an organic conductive compound is used, a wet film formation method such as a printing method or a coating method may be used. For taking emitted light from the anode, it is desired to have a transmittance of more than 10%, and the sheet resistance for the anode is preferably several hundreds Ω/□ or less. The film thickness is selected usually within 10 to 1000 nm, preferably within 10 to 200 nm though depending on the material.

### - Cathode -

Meanwhile, regarding a cathode material, a material of a metal (an electron injection metal), an alloy, an electrically conductive compound, or a mixture thereof, each having a small work function (4 eV or less) is used. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide (Al₂O₃) mixture, indium, a lithium/aluminum mixture, and a rare earth metal. Among these, from the viewpoint of the electron injectability and the durability against oxidation and the like, a mixture of an electron injection metal and a second metal which is a stable metal having a larger work function value is suitable, and examples thereof include a magnesium/silver mixture, a magnesium/aluminum mixture, a magnesium/indium mixture, an aluminum/aluminum oxide mixture, a lithium/aluminum mixture and aluminum. The cathode can be produced by forming a thin film by a method such as vapor-depositing or sputtering of such a cathode material. In addition, the sheet resistance of cathode is preferably several hundreds Ω/□ or less. The film thickness is selected usually within 10 nm to 5 µm, preferably within 50 to 200 nm. Note that for transmission of emitted light, if either one of the anode and cathode of the organic EL device is transparent or translucent, emission luminance is improved, which is convenient.

In addition, formation of a film of the above metal with a thickness of 1 to 20 nm, followed by formation of a conductive transparent material described in the description on the anode thereon, enables production of a transparent or translucent cathode, and application of this enables production of a device wherein an anode and a cathode both have transmittance.

### - Light-emitting layer -

The light-emitting layer is a layer that emits light after excitons are generated when holes and electrons injected from the anode and the cathode, respectively, are recombined. As a light-emitting layer, an organic light-emitting dopant material and a host may be contained.

As a host, the compound for an organic EL device of the present invention may be used.

Regarding the compound of the present invention as the host, one kind thereof may be used, or two or more kinds of different compounds may be used. If necessary, one, or two or more other known host materials may be used in combination. Such another host material is preferably a compound having hole transport ability and electron transport ability, preventing an extension in wavelength of light emitted, and having a high glass transition temperature.

Such known host materials are those known by many Patent Literatures and the like, and can be selected therefrom. Such host materials are not particularly limited, and specific examples thereof include indolocarbazole derivatives described in WO2008/056746A, WO2008/146839A, and the like, carbazole derivatives described in WO 2009/086028A, WO2012/077520A, and the like, CBP (N,N-biscarbazolylbiphenyl) derivatives, triazine derivatives described in WO2014/185595A, WO2018/021663A, and the like, indenocarbazole derivatives described in WO2010/136109A, WO2011/000455A, and the like, dibenzofuran derivatives described in WO 2015/169412A and the like, triazole derivatives, indole derivatives, oxazole derivatives, oxadiazole derivatives, imidazole derivatives, polyarylalkane derivatives, pyrazoline derivatives, pyrazolone derivatives, phenylenediamine derivatives, arylamine derivatives, amino-substituted chalcone derivatives, styryl anthracene derivatives, fluorenone derivatives, hydrazone derivatives, stilbene derivatives, silazane derivatives, aromatic tert-amine compounds, styrylamine compounds, aromatic dimethylidene-based compounds, porphyrin-based compounds, anthraquinodimethane derivatives, anthrone derivatives, diphenylquinone derivatives, thiopyran dioxide derivatives, heterocyclic tetracarboxylic anhydride such as naphthalene perylene, metal complexes such as phthalocyanine derivatives and 8-quinolinol derivatives, various metal complexes typified by metal complexes such as metal phthalocyanine, benzoxazole and benzothiazole derivatives, and polymer compounds such as polysilane-based compounds, poly(N-vinylcarbazole) derivatives, aniline-based copolymers, thiophene oligomers, polythiophene derivatives, polyphenylene derivatives, polyphenylene vinylene derivatives, and polyfluorene derivatives.

Specific examples of such known host materials are shown below, but are not limited thereto.

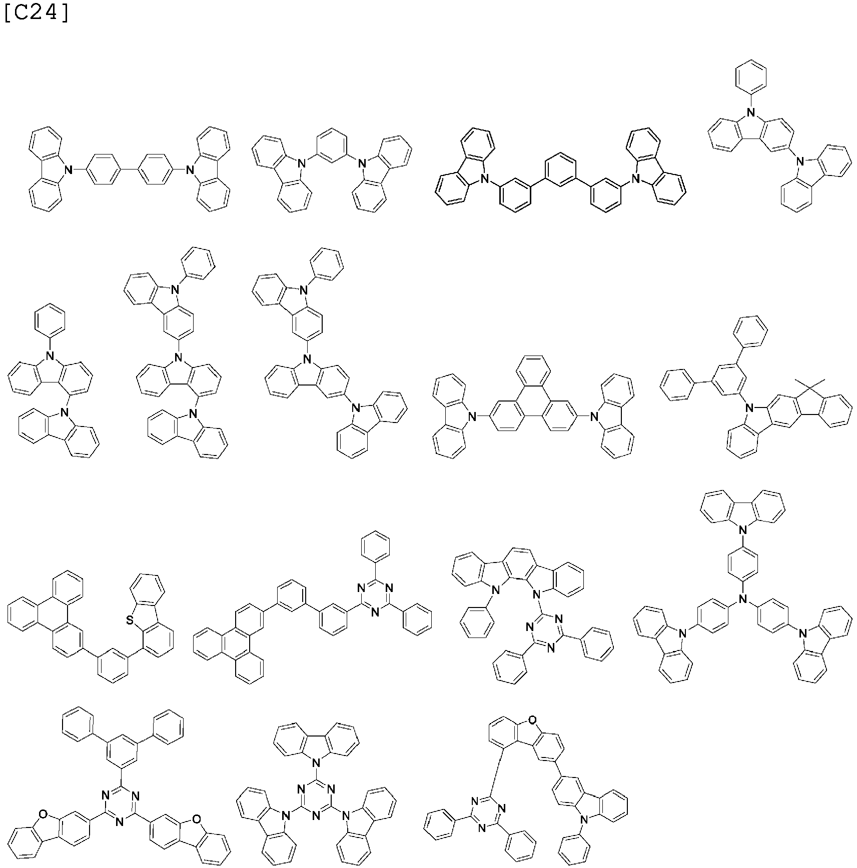

When a phosphorescent dopant is used as a light-emitting dopant material, preferred is a phosphorescent dopant including an organic metal complex containing at least one metal selected from ruthenium, rhodium, palladium, silver, rhenium, osmium, iridium, platinum and gold. Specifically, iridium complexes described in J. Am. Chem. Soc. 2001, 123, 4304, JP2013-530515A, US2016/0049599A, US2017/0069848A, US2018/0282356A, US2019/0036043A, and the like, or platinum complexes described in US2018/0013078A, KR2018-094482A, and the like are preferably used, but the phosphorescent dopant is not limited thereto.

Regarding the phosphorescent dopant material, only one kind thereof may be contained in the light-emitting layer, or two or more kinds thereof may be contained. A content of the phosphorescent dopant material is preferably 0.1 to 30 wt% and more preferably 1 to 20 wt% with respect to the host material.

The phosphorescent dopant material is not particularly limited, and specific examples thereof include the following.

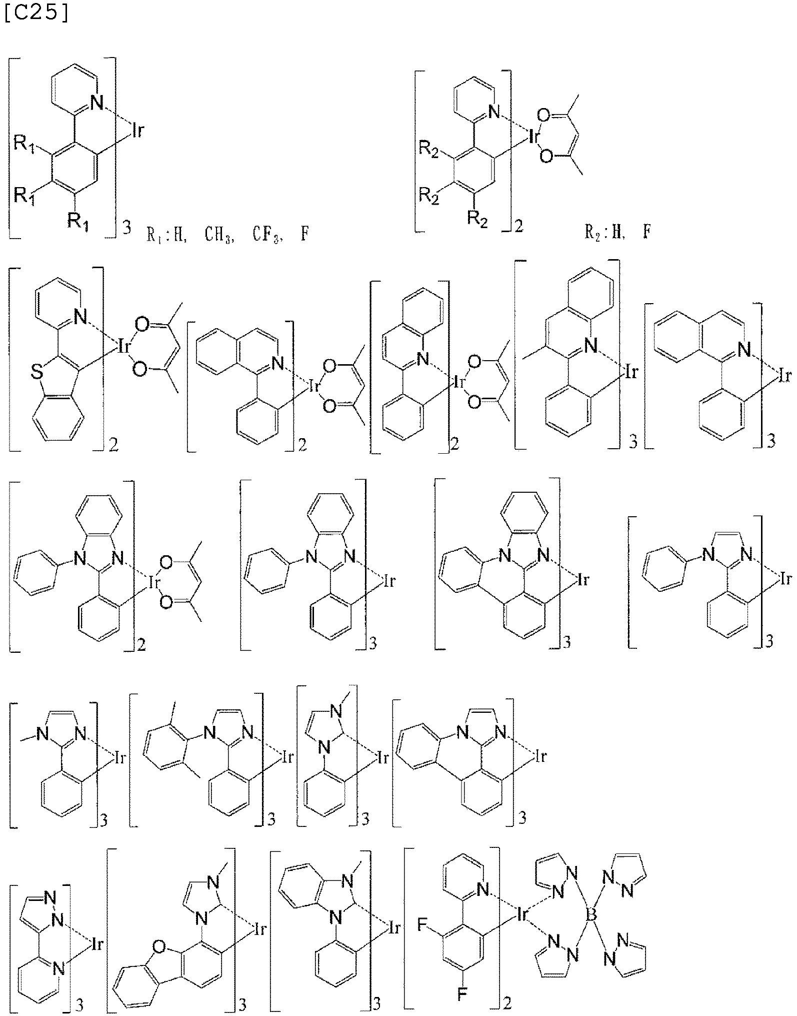

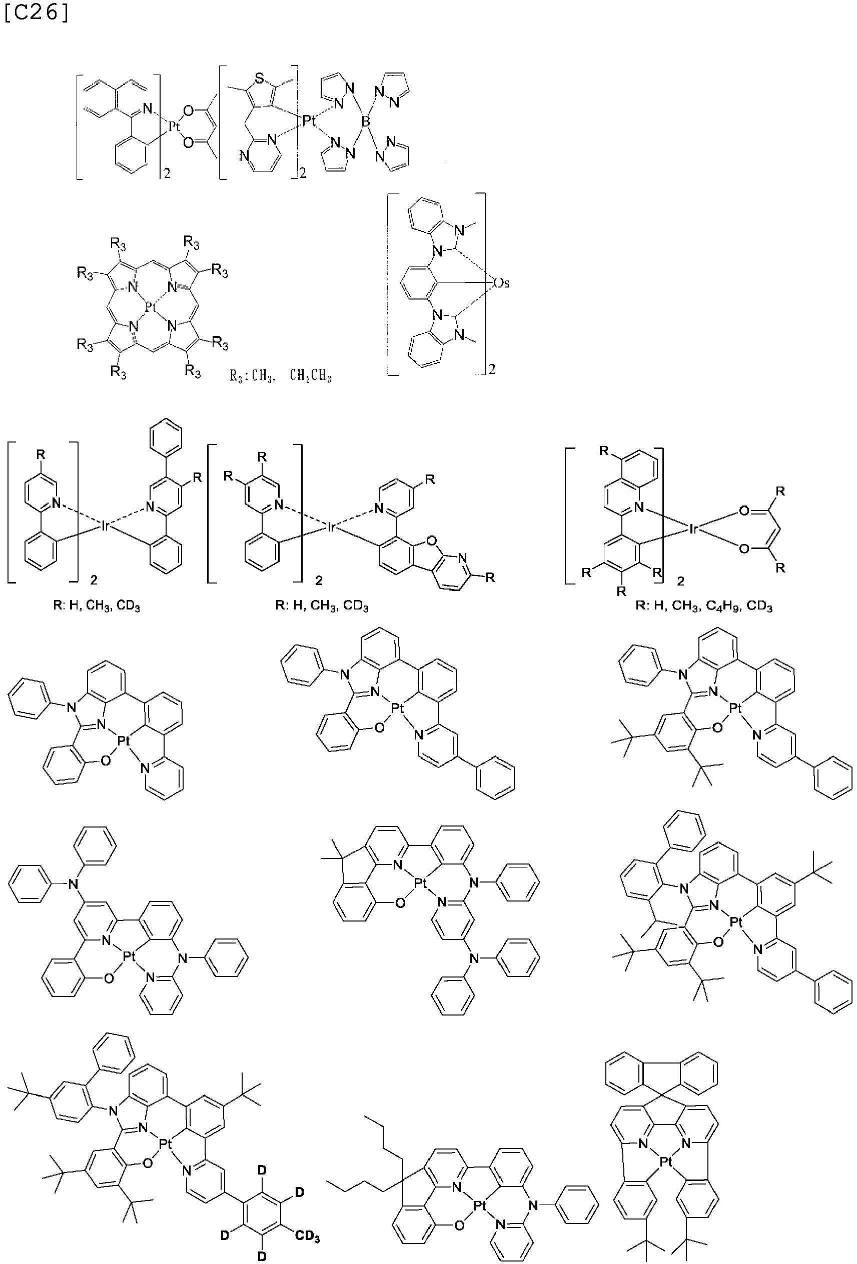

When a fluorescence-emitting dopant is used as the light-emitting dopant material, the fluorescence-emitting dopant is not particularly limited. Examples thereof include benzoxazole derivatives, benzothiazole derivatives, benzimidazole derivatives, styrylbenzene derivatives, polyphenyl derivatives, diphenylbutadiene derivatives, tetraphenyl butadiene derivatives, naphthalimido derivatives, coumarin derivatives, fused aromatic compounds, perinone derivatives, oxadiazole derivatives, oxazine derivatives, aldazine derivatives, pyrrolidine derivatives, cyclopentadiene derivatives, bisstyryl anthracene derivatives, quinacridone derivatives, pyrrolopyridine derivatives, thiadiazolopyridine derivatives, styrylamine derivatives, diketopyrrolopyrrole derivatives, aromatic dimethylidine compounds, metal complexes of 8-quinolinol derivatives or metal complexes of pyromethene derivatives, rare earth complexes, various metal complexes represented by transition metal complexes, polymer compounds such as polythiophene, polyphenylene, and polyphenylene vinylene, and organosilane derivatives. Preferred examples thereof include fused aromatic derivatives, styryl derivatives, diketopyrrolopyrrole derivatives, oxazine derivatives, pyromethene metal complexes, transition metal complexes, and lanthanoid complexes. More preferable examples thereof include naphthalene, pyrene, chrysene, triphenylene, benzo[c]phenanthrene, benzo[a]anthracene, pentacene, perylene, fluoranthene, acenaphthofluoranthene, dibenzo[a,j]anthracene, dibenzo[a,h]anthracene, benzo[a]naphthalene, hexacene, naphtho[2,1-f]isoquinoline, α-naphthaphenanthridine, phenanthrooxazole, quinolino[6,5-f]quinoline, and benzothiophanthrene. These may have an alkyl group, an aryl group, an aromatic heterocyclic group, or a diarylamino group as a substituent.

Regarding the fluorescence-emitting dopant material, only one kind thereof may be contained in the light-emitting layer, or two or more kinds thereof may be contained. A content of the fluorescence-emitting dopant material is preferably 0.1% to 20% and more preferably 1% to 10% with respect to the host material.

When a thermally activated delayed fluorescence-emitting dopant is used as the light-emitting dopant material, the thermally activated delayed fluorescence-emitting dopant is not particularly limited. Examples thereof include: metal complexes such as a tin complex and a copper complex; indolocarbazole derivatives described in WO2011/070963 A; cyanobenzene derivatives and carbazole derivatives described in Nature 2012, 492, 234; and phenazine derivatives, oxadiazole derivatives, triazole derivatives, sulfone derivatives, phenoxazine derivatives, and acridine derivatives described in Nature Photonics 2014, 8,326.

The thermally activated delayed fluorescence-emitting dopant material is not particularly limited, and specific examples thereof include the following.

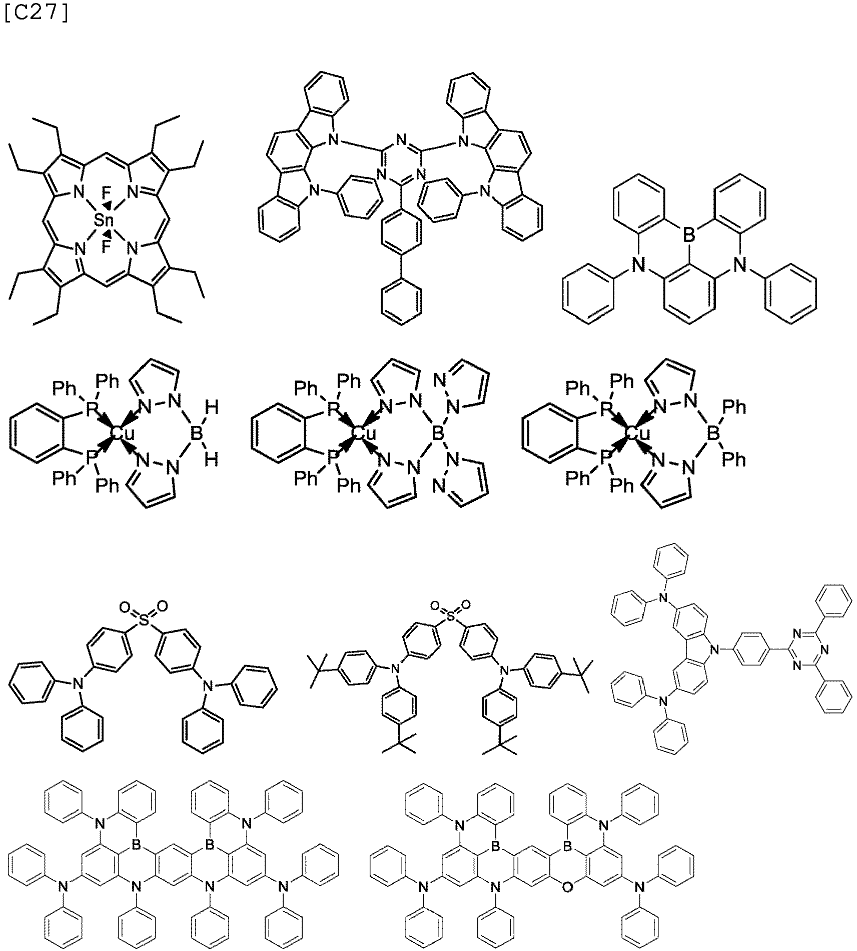

Regarding the thermally activated delayed fluorescence-emitting dopant material, only one kind thereof may be contained in the light-emitting layer, or two or more kinds thereof may be contained. In addition, the thermally activated delayed fluorescence-emitting dopant may be used by mixing with a phosphorescent dopant and a fluorescence-emitting dopant. A content of the thermally activated delayed fluorescence-emitting dopant material is preferably 0.1% to 50% and more preferably 1% to 30% with respect to the host material.

### - Injection Layer -

The injection layer is a layer that is provided between an electrode and an organic layer in order to lower a driving voltage and improve emission luminance, and includes a hole injection layer and an electron injection layer, and may be present between the anode and the light-emitting layer or the hole transport layer, and between the cathode and the light-emitting layer or the electron transport layer. The injection layer can be provided as necessary.

### - Hole Blocking Layer -

The hole blocking layer has a function of the electron transport layer in a broad sense, and is made of a hole blocking material having a function of transporting electrons and a significantly low ability to transport holes, and can block holes while transporting electrons, thereby improving a probability of recombining electrons and holes in the light-emitting layer.

### - Electron Blocking Layer -

The electron blocking layer has a function of a hole transport layer in a broad sense and blocks electrons while transporting holes, thereby enabling a probability of recombining electrons and holes in the light-emitting layer to be improved.

Regarding the material of the electron blocking layer, a known electron blocking layer material can be used and a material of the hole transport layer to be described below can be used as necessary. A film thickness of the electron blocking layer is preferably 3 to 100 nm, and more preferably 5 to 30 nm.

### - Exciton Blocking Layer -

The exciton blocking layer is a layer for preventing excitons generated by recombination of holes and electrons in the light-emitting layer from being diffused in a charge transport layer, and insertion of this layer allows excitons to be efficiently confined in the light-emitting layer, enabling the luminous efficiency of the device to be improved. The exciton blocking layer can be inserted, in a device having two or more light-emitting layers adjacent to each other, between two adjacent light-emitting layers.

Regarding the material of the exciton blocking layer, a known exciton blocking layer material can be used. Examples thereof include 1,3-dicarbazolyl benzene (mCP) and bis(2-methyl-8-quinolinolato)-4-phenylphenolato aluminum (III) (BAlq).

### - Hole Transport Layer -

The hole transport layer is made of a hole transport material having a function of transporting holes, and the hole transport layer can be provided as a single layer or a plurality of layers.

The hole transport material has either hole injection, transport properties or electron barrier properties, and may be an organic material or an inorganic material. For the hole transport layer, any one selected from conventionally known compounds can be used. Examples of such a hole transport material include porphyrin derivatives, arylamine derivatives, triazole derivatives, oxadiazole derivatives, imidazole derivatives, polyarylalkane derivatives, pyrazoline derivatives and pyrazolone derivatives, phenylenediamine derivatives, arylamine derivatives, amino-substituted chalcone derivatives, oxazole derivatives, styryl anthracene derivatives, fluorenone derivatives, hydrazone derivatives, stilbene derivatives, silazane derivatives, an aniline copolymer, and a conductive polymer oligomer, and particularly a thiophene oligomer. Use of porphyrin derivatives, arylamine derivatives, or styrylamine derivatives is preferred. Use of arylamine compounds is more preferred.

### - Electron Transport Layer -

The electron transport layer is made of a material having a function of transporting electrons, and the electron transport layer can be provided as a single layer or a plurality of layers.

The electron transport material (which may also serve as a hole blocking material) may have a function of transferring electrons injected from the cathode to the light-emitting layer. For the electron transport layer, any one selected from conventionally known compounds can be used, and examples thereof include polycyclic aromatic derivatives such as naphthalene, anthracene, and phenanthroline, tris(8-quinolinolato)aluminum(III) derivatives, phosphine oxide derivatives, nitrosubstituted fluorene derivatives, diphenylquinone derivatives, thiopyrandioxide derivatives, carbodiimide, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. In addition, a polymer material in which the above material is introduced into a polymer chain or the above material is used for a main chain of a polymer can be used.

### Examples

Hereafter, the present invention will be described in detail by referring to Examples, but the present invention is not limited these Examples and can be implemented in various forms without departing from the gist thereof.

### Example 1

Compound (2) was synthesized in accordance with the next reaction formula.

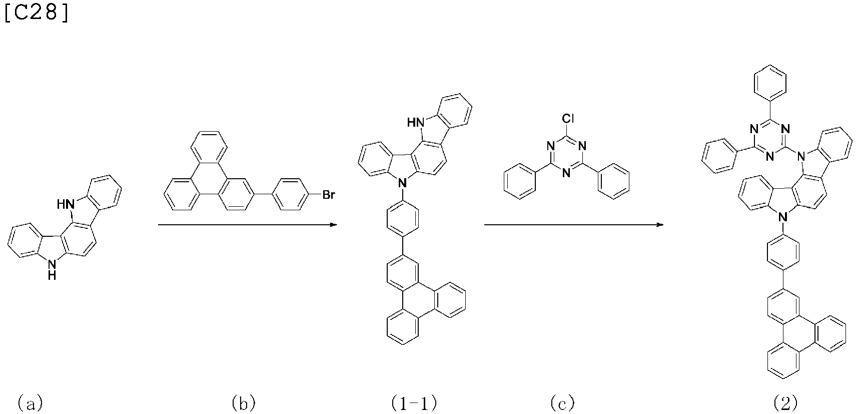

To 20 g of compound (a) were added 36 g of compound (b), 1.5 g of copper iodide, 43 g of potassium carbonate, 0.06 g of 18-crown-6-ether, and 900 ml of 1,3-dimethyl-2-imidazolidinone, and the mixture was stirred under a nitrogen atmosphere at 190°C for 19 hours. The mixture was cooled to room temperature, and then purified by silica gel column chromatography and crystallization to give 31 g (71% yield) of intermediate (1-1) as a white solid.

Under a nitrogen atmosphere, 1.4 g of 60 wt% sodium hydride was added to 30 ml of N,N'-dimethylacetamide (DMAc) to prepare suspension. To this was added 17 g of intermediate (1-1) dissolved in 170 mL of DMAc, then the mixture was stirred for 30 minutes. To this was added 7.9 g of compound (c), then the mixture was stirred for 4 hours. The reaction solution was added to a solution of methanol (300 ml) mixed with distilled water (100 ml) while being stirred, and the resulting precipitated solid was collected by filtration. The resulting solid was purified by silica gel chromatography and crystallization to give 20 g (70% yield) of compound (2) as a yellow solid compound (APCI-TOFMS, m/z 790 [M+H]⁺).

### Example 2

Compound (81) was synthesized in accordance with the next reaction formula.

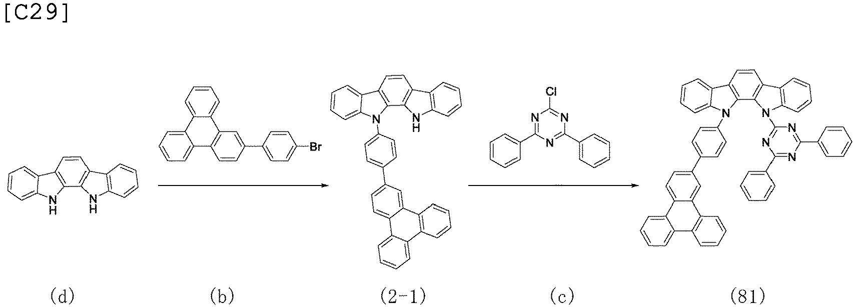

To 20 g of compound (d) were added 36 g of compound (b), 1.5 g of copper iodide, 43 g of potassium carbonate, 0.06 g of 18-crown-6-ether, and 900 ml of 1,3-dimethyl-2-imidazolidinone, and the mixture was stirred for 72 hours. The reaction product was separated and purified to give 32 g (73% yield) of intermediate (2-1) as a white solid.

To 30 ml of DMAc was added 1.4 g of 60 wt% sodium hydride, 17 g of intermediate (2-1) dissolved in DMAc was added thereto, and the mixture was stirred for 30 minutes. To this was added 7.9 g of compound (c), then the mixture was stirred for 24 hours. The reaction product was separated and purified to give 18 g (76% yield) of compound (81) as a yellow solid compound.

### Example 3

Compound (82) was synthesized in accordance with the next reaction formula.

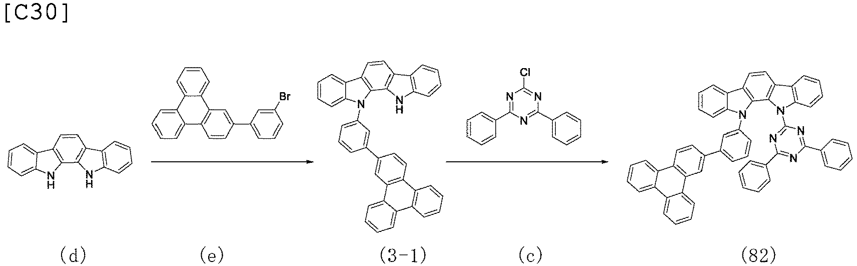

To 20 g of compound (d) were added 36 g of compound (e), 1.5 g of copper iodide, 43 g of potassium carbonate, 0.06 g of 18-crown-6-ether, and 900 ml of 1,3-dimethyl-2-imidazolidinone, and the mixture was stirred for 72 hours. The reaction product was separated and purified to give 28 g (64% yield) of intermediate (3-1) as a white solid.

To 30 ml of DMAc was added 1.4 g of 60 wt% sodium hydride, 17 g of intermediate (3-1) dissolved in DMAc was added thereto, and the mixture was stirred for 30 minutes. To this was added 7.9 g of compound (c), then the mixture was stirred for 4 hours. The reaction product was separated and purified to give 21 g (89% yield) of compound (82) as a yellow solid compound.

### Example 4

Compound (83) was synthesized in accordance with the next reaction formula.

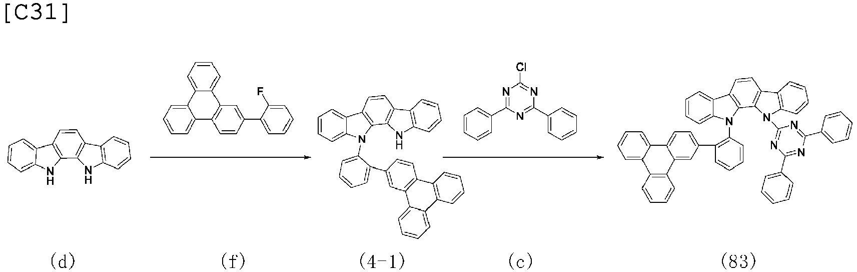

To 10.8 g of compound (d) were added 15 g of compound (f), 36 g of tripotassium phosphate, 2.8 g of 18-crown-6-ether, and 180 ml of 1,3-dimethyl-2-imidazolidinone, and the mixture was stirred for 90 hours. The reaction product was separated and purified to give 25 g (57% yield) of intermediate (4-1) as a white solid.

To 20 ml of DMAc was added 0.6 g of 60 wt% sodium hydride, 9 g of intermediate (4-1) dissolved in DMAc was added thereto, and the mixture was stirred for 30 minutes. To this was added 4.6 g of compound (c), then the mixture was stirred for 27 hours. The reaction product was separated and purified to give 9 g (70% yield) of compound (83) as a yellow solid compound.

### Example 5

Compound (85) was synthesized in accordance with the next reaction formula.

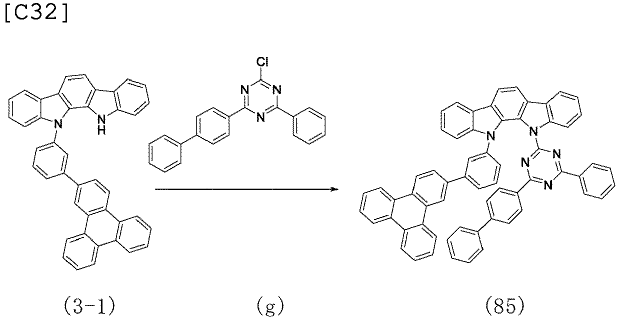

To 30 ml of DMAc was added 1.4 g of 60 wt% sodium hydride, 11 g of intermediate (3-1) dissolved in DMAc was added thereto, and the mixture was stirred for 30 minutes. To this was added 7.9 g of compound (g), then the mixture was stirred for 24 hours. The reaction product was separated and purified to give 19 g (73% yield) of compound (85) as a yellow solid compound.

### Example 6

Compound (93) was synthesized in accordance with the next reaction formula.

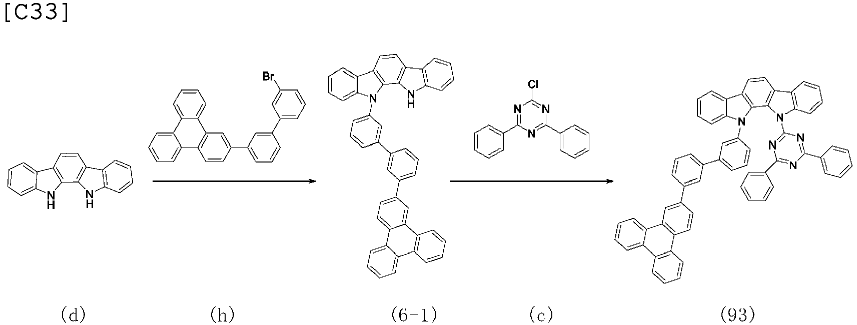

To 20 g of compound (d) were added 43 g of compound (h), 1.5 g of copper iodide, 43 g of potassium carbonate, 0.06 g of 18-crown-6-ether, and 900 ml of 1,3-dimethyl-2-imidazolidinone, and the mixture was stirred for 72 hours. The reaction product was separated and purified to give 37 g (75% yield) of intermediate (6-1) as a white solid.

To 30 ml of DMAc was added 1.4 g of 60 wt% sodium hydride, 11 g of intermediate (6-1) dissolved in DMAc was added thereto, and the mixture was stirred for 30 minutes. To this was added 7.9 g of compound (c), then the mixture was stirred for 72 hours. The reaction product was separated and purified to give 22 g (86% yield) of compound (93) as a yellow solid compound.

### Example 7

Compound (103) was synthesized in accordance with the next reaction formula.

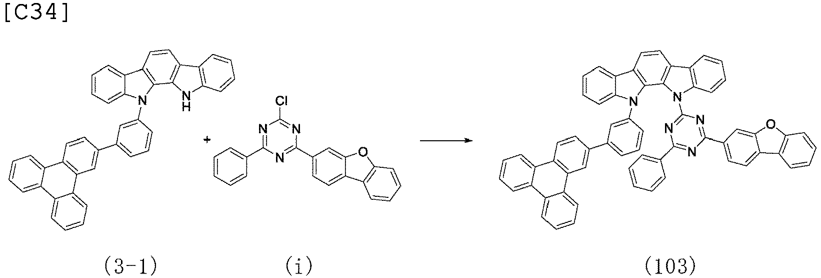

To 30 ml of DMAc was added 1.4 g of 60 wt% sodium hydride, 11 g of intermediate (3-1) dissolved in DMAc was added thereto, and the mixture was stirred for 30 minutes. To this was added 11 g of compound (i), then the mixture was stirred for 72 hours. The reaction product was separated and purified to give 19 g (72% yield) of compound (103) as a yellow solid compound.

### Example 8

On a glass substrate on which an anode made of ITO with a film thickness of 110 nm was formed, respective thin films were laminated by a vacuum evaporation method at a degree of vacuum of 4.0 × 10⁻⁵ Pa. First, HAT-CN was formed with a thickness of 25 nm as a hole injection layer on ITO, and next, Spiro-TPD was formed with a thickness of 30 nm as a hole transport layer. Next, HT-1 was formed with a thickness of 10 nm as an electron blocking layer. Then, compound (2) as a host material and Ir(ppy)₃ as a light-emitting dopant were co-vapor-deposited from different vapor deposition sources, respectively, to form a light-emitting layer with a thickness of 40 nm. In this case, the concentration of Ir(ppy)₃ was 10 wt%. Next, ET-1 was formed with a thickness of 20 nm as an electron transport layer. Further, LiF was formed with a thickness of 1 nm as an electron injection layer on the electron transport layer. Finally, Al was formed with a thickness of 70 nm as a cathode on the electron injection layer to produce an organic EL device.

### Examples 9 to 14

Organic EL devices were produced in the same manner as in Example 8 except that compounds (81), (82), (83), (85), (93), and (103) obtained in Examples 2 to 7 were used as host materials in light-emitting layers. Table 3 shows light-emitting characteristics.

### Comparative Examples 1 to 6

Organic EL devices were produced in the same manner as in Example 8 except that compounds A, B, C, D, E, or F were used as the host material of the light-emitting layer instead of that of Example 8.

When an external power supply was connected to the organic EL devices obtained in Examples and Comparative Examples and a DC voltage was applied, an emission spectrum with a maximum wavelength of 517 nm was observed in all the organic EL devices and it was thus found that light emission from Ir(ppy)₃ was obtained.

Evaluation results of the produced organic EL devices are shown in Table 1. In the table, the luminance, driving voltage, power efficiency, and LT70 are values at a driving current of 20 mA/cm². LT70 is a time period needed for the initial luminance to be reduced from 9000 cd/A to 70% thereof, and it represents lifetime characteristics.

**[Table 1]**

| | Host compound | Voltage (V) | Luminance (cd/m²) | Power efficiency (Im/W) | LT70 (h) |
|---|---|---|---|---|---|
| Example 8 | 2 | 3.9 | 10337 | 41.5 | 819 |
| Example 9 | 81 | 3.5 | 9631 | 43.6 | 726 |
| Example 10 | 82 | 3.7 | 9514 | 40.6 | 825 |
| Example 11 | 83 | 3.6 | 9647 | 41.6 | 792 |
| Example 12 | 85 | 3.6 | 9578 | 41.9 | 1056 |
| Example 13 | 93 | 3.5 | 9583 | 43.3 | 809 |
| Example 14 | 103 | 3.3 | 9782 | 46.7 | 611 |
| Comp. Example 1 | A | 3.6 | 9008 | 39.7 | 430 |
| Comp. Example 2 | B | 3.9 | 9845 | 39.4 | 426 |
| Comp. Example 3 | C | 4.4 | 9483 | 33.5 | 462 |
| Comp. Example 4 | D | 4.6 | 11236 | 38.5 | 306 |
| Comp. Example 5 | E | 3.7 | 8809 | 37.4 | 513 |
| Comp. Example 6 | F | 6.5 | 6705 | 16.1 | 229 |

From Table 1, it is understood that Examples 8 to 14 improved the power efficiency and the lifetime and exhibited good characteristics, as compared with Comparative Examples.

Compounds used in Examples and Comparative Examples are shown below.

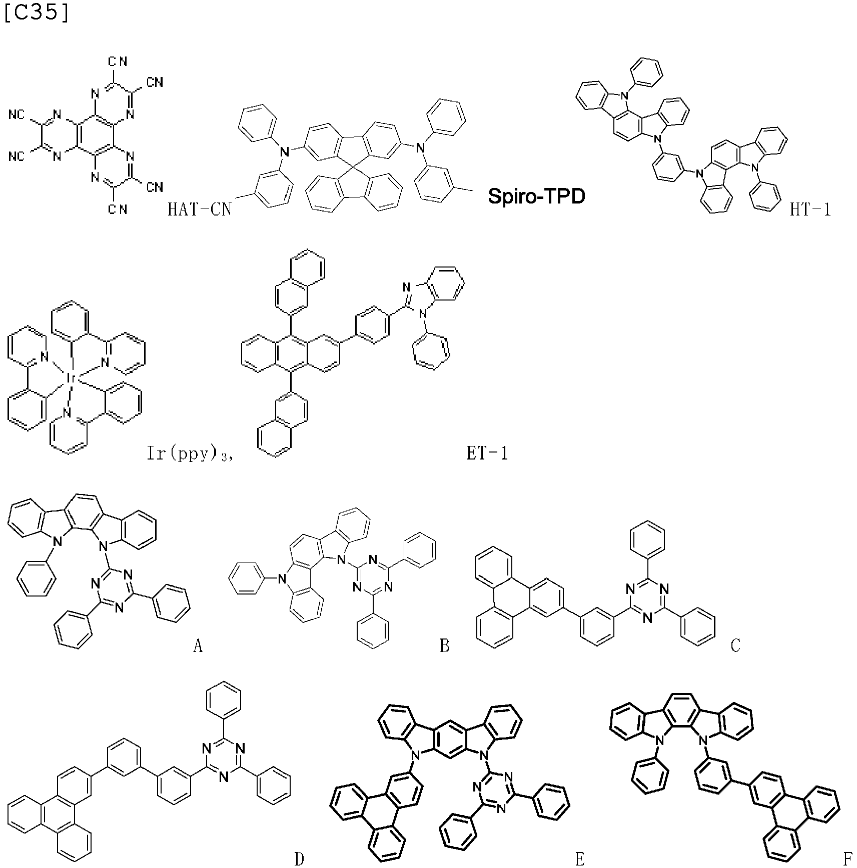

### Reference Signs List

1; substrate, 2; anode, 3; hole injection layer, 4; hole transport layer, 5; light-emitting layer, 6; electron transport layer, 7; cathode

## Claims

1. A compound for an organic electroluminescent device, represented by the following general formula (1): 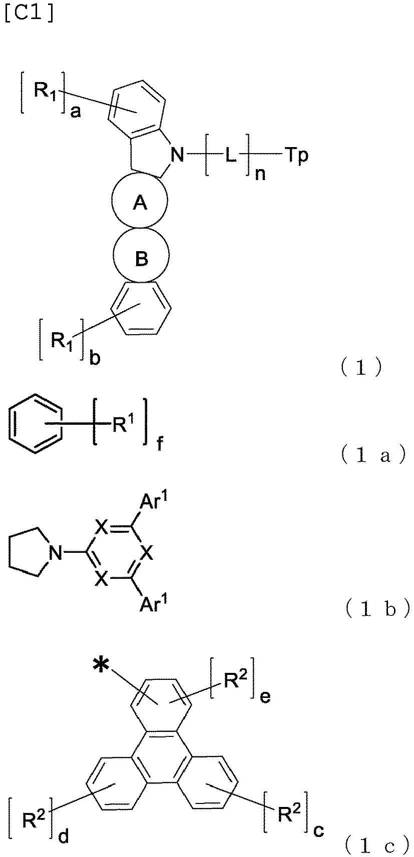 wherein a ring A is an aromatic ring represented by formula (1a), a ring B is a heterocycle represented by formula (1b), Tp represents a triphenylene group represented by formula (1c), and * represents a position for binding to L,
each R¹ independently represents deuterium or an aliphatic hydrocarbon group having 1 to 10 carbon atoms, L represents a substituted or unsubstituted aromatic hydrocarbon group having 6 to 18 carbon atoms,
each X independently represents N or C-H and at least one thereof represents N,
each Ar¹ independently represents hydrogen, a substituted or unsubstituted aromatic hydrocarbon group having 6 to 18 carbon atoms, a substituted or unsubstituted aromatic heterocyclic group having 3 to 17 carbon atoms, or a substituted or unsubstituted linked aromatic group in which two to five of these aromatic rings are linked to each other,
each R² independently represents deuterium, an aliphatic hydrocarbon group having 1 to 10 carbon atoms, a substituted or unsubstituted aromatic hydrocarbon group having 6 to 10 carbon atoms, a substituted or
unsubstituted aromatic heterocyclic group having 3 to 12 carbon atoms, or a substituted or unsubstituted linked aromatic group in which two to five aromatic rings of aromatic groups selected from the aromatic hydrocarbon group and the aromatic heterocyclic group are linked to each other, and
a to f represent the number of substitutions, a to d represent an integer of 0 to 4, e represents an integer of 0 to 3, f represents an integer of 0 to 2, and n represents the number of repetitions and an integer of 1 to 3.

2. The compound for an organic electroluminescent device according to claim 1, wherein all of X represent N.

3. The compound for an organic electroluminescent device according to claim 1 or 2, wherein L represents a phenylene group.

4. The compound for an organic electroluminescent device according to any one of claims 1 to 3, wherein n represents 1.

5. The compound for an organic electroluminescent device according to any one of claims 1 to 4, wherein all of a to f represent 0.

6. The compound for an organic electroluminescent device according to any one of claims 1 to 5, wherein the compound represented by the general formula (1) is represented by any one of the following formulas (2) to (5) : 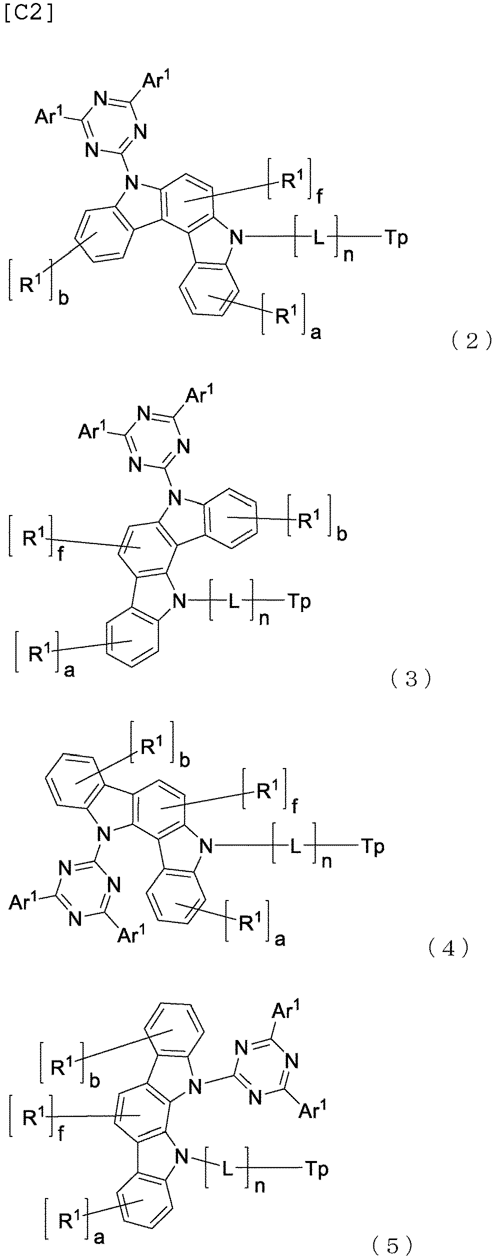 wherein Tp, Ar¹, L, R¹, R², a to f, and n are as defined for the general formula (1).

7. The compound for an organic electroluminescent device according to any one of claims 1 to 6, wherein Tp in the general formula (1) and the formulas (2) to (5) is represented by the following formula (2c): 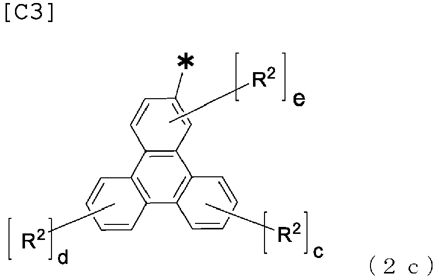 wherein R², c, d, e and * are as defined for the general formula (1).

8. An organic electroluminescent device having an organic layer between an anode and a cathode laminated on a substrate, wherein the organic layer contains the compound for an organic electroluminescent device according to any one of claims 1 to 7.

9. The organic electroluminescent device according to claim 8, wherein the organic layer is a light-emitting layer, and the light-emitting layer contains a light-emitting dopant and the compound for an organic electroluminescent device according to any one of claims 1 to 7 as a host material.
